(19) Europäisches Patentamt
European Patent Office
Office européen des brevets

(11) EP 2 516 398 B1

(12) **FASCICULE DE BREVET EUROPEEN**

(45) Date de publication et mention de la délivrance du brevet:
**21.03.2018 Bulletin 2018/12**

(21) Numéro de dépôt: **10808915.2**

(22) Date de dépôt: **22.12.2010**

(51) Int Cl.:
*C07D 213/74* (2006.01) *C07D 239/42* (2006.01)
*C07D 241/20* (2006.01) *A61K 31/44* (2006.01)
*A61K 31/4965* (2006.01) *A61K 31/505* (2006.01)
*A61K 8/49* (2006.01) *A61Q 19/00* (2006.01)
*A61P 17/00* (2006.01) *A61P 35/00* (2006.01)
*A61P 15/00* (2006.01)

(86) Numéro de dépôt international:
**PCT/FR2010/052871**

(87) Numéro de publication internationale:
**WO 2011/077043 (30.06.2011 Gazette 2011/26)**

(54) **DERIVES PHENOLIQUES, ET LEUR UTILISATION PHARMACEUTIQUE OU COSMETIQUE**

PHENOLDERIVATE UND DEREN PHARMAZEUTISCHE ODER KOSMETISCHE VERWENDUNG

PHENOLIC DERIVATIVES, AND THEIR PHARMACEUTICAL OR COSMETIC USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **23.12.2009 US 282153 P**
**23.12.2009 FR 0959475**

(43) Date de publication de la demande:
**31.10.2012 Bulletin 2012/44**

(73) Titulaire: **Galderma Research & Development**
**06410 Biot (FR)**

(72) Inventeurs:
- **POINSARD, Cédric**
**F-06130 Le Plan de Grasse (FR)**
- **COLLETTE, Pascal**
**F-06110 Le Cannet (FR)**
- **MAUVAIS, Pascale**
**F-06600 Antibes (FR)**
- **LINGET, Jean-Michel**
**F-67230 Benfeld (FR)**
- **RETHORE, Sandrine**
**F-06560 Valbonne (FR)**

(74) Mandataire: **Sekhri, Redha et al**
**Cabinet Becker & Associés**
**25, rue Louis le Grand**
**75002 Paris (FR)**

(56) Documents cités:
**WO-A1-2004/052868 WO-A1-2005/042464**
**US-A- 4 578 390**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 516 398 B1

## Description

La présente invention a pour objet de nouveaux composés de formule générale :

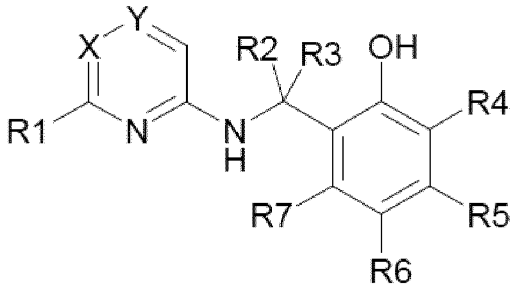

(I)

Et leur utilisation cosmétique ou pharmaceutique.

La présente invention se propose de fournir de nouveaux dérivés phénoliques, qui soient de puissants modulateurs du récepteur aux androgènes.

Parmi les documents de l'art antérieur décrivant des molécules modulant l'activité du récepteur aux androgènes, on peut, par exemple, citer les phenylimidazolines décrites dans la demande de brevet EP580459, ou la demande WO200542464.

US 4,578,390 décrit des dérivés hydroxybenzylamino en tant qu'agents anti-inflammatoires, et notamment des dérivés 1-amino-pyridiles dont la fonction amino est substituée par un groupe 2-hydroxybenzyle.

WO 2004/052868 décrit des dérivés de pyrazine en tant qu'inhibiteurs de la tubuline, et notamment leurs utilisations dans le traitement du cancer et des maladies prolifératives.

La présente invention a pour objet les revendications 1 à 11.

L'invention a pour objet de nouveaux dérivés phénoliques qui répondent à la formule générale (I) suivante :

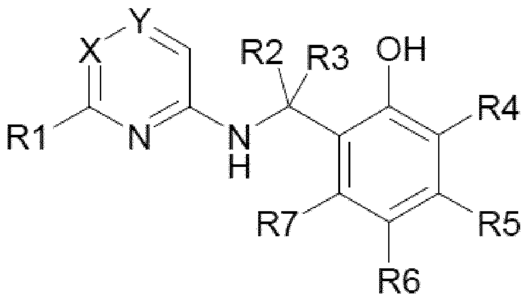

(I)

dans laquelle :

- $R_1$ représente un groupe $C_{2-6}$ alkyle, $C_{3-7}$ cycloalkyle, $C_{1-6}$ alkyloxy, $-S(O)_m-C_{1-6}$ alkyle, $C_{1-6}$ fluoroalkyle, $C_{1-6}$ fluoroalkyloxy, $-(CH_2)_n-C_{3-9}$ cycloalkyle, $C_{2-6}$ alkyle-OH,$-(CH_2)_n-C_{1-6}$ alkyloxy, $-(CH_2)_n-C_{1-6}$ fluoroalkyle, - $(CH_2)_p$-O-$C_{1-6}$ fluoroalkyle, $COR_a$, CN, $NO_2$, $NR_8R_9$, un halogène, un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents,
- $R_2$ et $R_3$ sont identiques ou différents et représentent un atome d'hydrogène ou un groupe $C_{1-9}$ alkyle, $C_{3-9}$ cycloalkyle, $C_{1-6}$ fluoroalkyle, $-(CH_2)_r-C_{3-9}$ cycloalkyle, $-C_{2-6}$ alkyle-OH, $-(CH_2)_r-C_{1-6}$ alkyloxy, $-(CH_2)_r-C_{3-7}$ cycloalkyle, $-(CH_2)_r-C_{1-6}$ fluoroalkyle,$-(CH_2)_q-O-C_{1-6}$ fluoroalkyle
  Eventuellement les groupes $R_2$ et $R_3$ peuvent former avec l'atome de carbone qui les porte un groupe $C_{3-9}$ cycloalkyle ou un hétérocycle tel que tetrahydrofuranne, tetrahydropyranne, tetrahydrothiopyranne, tetrahydro-1 oxy-thiopyranne ou tetrahydro-1,1 dioxy thiopyranne.
- $R_4$, $R_5$, $R_6$, $R_7$ sont identiques ou différents et représentent soit un atome d'hydrogène soit un groupe $C_{1-6}$ alkyle, $C_{3-7}$ cycloalkyle, $C_{1-6}$ alkyloxy, $-S(O)_s-C_{1-6}$ alkyle, $C_{1-6}$ fluoroalkyle, $C_{1-6}$ fluoroalkyloxy, $-(CH_2)_t-C_{3-9}$ cycloalkyle,

$-(CH_2)_t-C_{3-9}$ cycloalkyle, $-C_{1-6}$ alkyle -OH, $-(CH_2)_t-C_{1-6}$ alkyloxy, $-(CH_2)_t-C_{1-6}$ fluoroalkyle, $-(CH_2)_u-O-C_{1-6}$ fluoroalkyle, $COR_d$, CN, $NR_{8'}R_{9'}$, ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_c$ identiques ou différents

- X représente CH ou N
- Y représente soit un atome d'azote, soit un atome de carbone substitué par un groupe $C_{1-6}$ alkyle, $C_{3-7}$ cycloalkyle, $C_{1-6}$ alkyloxy, $-S(O)_v-C_{1-6}$ alkyle, $C_{1-6}$ fluoroalkyle, $C_{1-6}$ fluoroalkyloxy, $-(CH_2)_l-C_{3-9}$ cycloalkyle, $-(CH_2)_l-C_{3-9}$ cycloalkyle, $C_{1-6}$ alkyle-OH, $-(CH_2)_l-C_{1-6}$ alkyloxy, $-(CH_2)_l-C_{1-6}$ fluoroalkyle, $-(CH_2)_w-O-C_{1-6}$ fluoroalkyle, $COR_e$, CN, $NR_{10}R_{11}$, $NO_2$, un atome d'hydrogène ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents
- $R_a$, $R_d$ et $R_e$ sont identiques ou différents et représentent un groupe $C_{1-6}$ alkyle, $C_{1-6}$ alkyloxy ou $NR_{12}R_{13}$,- $R_b$ et $R_c$ sont identiques ou différents et représentent un halogène, un groupe $C_{1-6}$ alkyle, $C_{3-7}$ cycloalkyle, $C_{1-6}$ alkyloxy, $-S(O)_j-C_{1-6}$ alkyle, $C_{1-6}$ fluoroalkyle, $C_{1-6}$ fluoroalkyloxy, $-(CH_2)_i-C_{3-7}$ cycloalkyle, OH, $-(CH_2)_i-C_{3-7}$ cycloalkyle, $C_{1-6}$ alkyle-OH, $-(CH_2)_i-C_{1-6}$ alkyloxy, $-(CH_2)_i-C_{1-6}$ fluoroalkyle, $-(CH_2)_z-O-C_{1-6}$ fluoroalkyle, $COR_a$, CN, ou $NR_{14}R_{15}$
- $R_8$ et $R_{8'}$ sont identiques ou différents et représentent un groupe $C_{1-6}$ alkyle, $C_{3-7}$ cycloalkyle, $-(CH_2)_f-C_{3-7}$ cycloalkyle ou $-(CH_2)_f-C_{1-6}$ fluoroalkyle.
- $R_9$, $R_{9'}$, $R_{10}$, $R_{11}$, $R_{12}$, $R_{13}$, $R_{14}$ et $R_{15}$ sont identiques ou différents et représentent un atome d'hydrogène, un groupe $C_{1-6}$ alkyle, $C_{3-7}$ cycloalkyle, $-(CH_2)_g-C_{3-7}$ cycloalkyle ou $-(CH_2)_g-C_{1-6}$ fluoroalkyle.

  Eventuellement les groupes $R_8$ et $R_9$ peuvent former avec l'atome d'azote qui les porte un hétérocycle tel que : azetidine, pyrolidine, pipéridine, azepane, morpholine ou pipérazine. Eventuellement les groupes $R_{8'}$ et $R_{9'}$ peuvent former avec l'atome d'azote qui les porte un hétérocycle tel que : azetidine, pyrolidine, pipéridine, azepane, morpholine ou pipérazine. Eventuellement les groupes $R_{10}$ et $R_{11}$ peuvent former avec l'atome d'azote qui les porte un hétérocycle tel que : azetidine, pyrolidine, pipéridine, azepane, morpholine ou pipérazine. Eventuellement les groupes $R_{12}$ et $R_{13}$ peuvent former avec l'atome d'azote qui les porte un hétérocycle tel que : azetidine, pyrolidine, pipéridine, azepane, morpholine ou pipérazine. Eventuellement les groupes $R_{14}$ et $R_{15}$ peuvent former avec l'atome d'azote qui les porte un hétérocycle tel que : azetidine, pyrolidine, pipéridine, azepane, morpholine ou pipérazine.
- f, g, i, l, n, r et t sont différents ou identiques et sont égales à 1, 2 ou 3
- j, m, s et v sont différents ou identiques et sont égales à 0, 1 ou 2
- p, q, u, w et z sont différents ou identiques et sont égales à 2, 3 ou 4

ainsi que leurs sels pharmaceutiquement acceptables, solvates ou hydrates et leurs conformères ou rotamères.

Les composés de formule (I) peuvent comporter un ou plusieurs atomes de carbone asymétriques. Ils peuvent donc exister sous forme de mélange d'énantiomères ou de diastéréoisomères. Ces énantiomères, diastéréoisomères ainsi que leurs mélanges y compris les mélanges racémiques font partie de l'invention.

Les composés de formule (I) peuvent exister à l'état de base ou de sels d'addition à des acides. De tels sels d'addition font partie de l'invention. Ces sels sont avantageusement préparés avec des acides pharmaceutiquement acceptables mais les sels d'autres acides utiles, par exemple pour la purification ou l'isolement des composés de formule (I) font également partie de l'invention. Ces acides peuvent être par exemple l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate. Pour une revue des sels physiologiquement acceptables voir Handbook of Pharmaceutical Salts : Properties, Selection and Use de Stahl etWermuth (Wiley-VCH, 2002).

Les solvates ou hydrates pourront être obtenus directement à l'issue du procédé de synthèse, le composé (I) étant isolé sous la forme d'un hydrate, par exemple un mono ou hémi-hydrate ou d'un solvate du solvant de réaction ou de purification.

Dans le cadre de l'invention on entend par :

- $C_{b-c}$ où b et c peuvent prendre des valeurs de 1 à 9, une chaine carbonnée de b à c atomes de carbone, par exemple $C_{1-6}$ une chaine carbonnée pouvant avoir 1 à 6 atomes de carbone
- alkyle, un groupe aliphatique saturé linéaire ou ramifié, par exemple un groupe $C_{1-6}$ alkyle représente une chaine carbonnée de 1 à 6 atomes de carbone, linéaire ou ramifiée, préférentiellement un méthyle, éthyle, propyle, isopropyle, butyle, isobutyle, terbutyle, pentyle, hexyle
- cycloalkyle, une chaine carbonnée saturée cyclique éventuellement ramifiée, comportant de 3 à 7 atomes de carbone. A titre d'exemple un groupe $C_{3-7}$ cycloalkyle représente une chaine carbonnée de 3 à 7 atomes de carbone préférentiellement un cyclopropyle, cyclobutyle, cyclopentyle, cyclohexyle et cycloheptyle
- hétérocycle, une chaine hydrocarbonée cyclique ou bicylique, saturée ou insaturée, comprenant un ou plusieurs

hétéroatomes choisis parmi O, S et N,

- heteroaryle, un hétérocycle aromatique, préférentiellement un groupe pyridinyle, pyrazinyle, pyrimidinyle, pyridazinyle, triazinyle, pyrazolyle, isooxazolyle, oxadiazolyle, thiazolyle, thiadiazolyle, triazolyle ou imidazolyle
- halogène, un atome de fluore, de chlore ou de brome
- alkyloxy, un groupe -O-alkyle
- alkylthio, un groupe -S-alkyle
- fluoroalkyle, un groupe alkyle dont un ou plusieurs atomes d'hydrogène ont été remplacés par un atome de fluor
- fluoroalkyloxy, un groupe alkyloxy dont un ou plusieurs atomes d'hydrogène ont été remplacés par un atome de fluor

Est préféré le groupe (A) des composés de formule (I) ci-dessus définis, dans lesquels :

- X représente CH et Y représente un atome de carbone substitué par un des groupes tels que définis ci-dessus et préférentiellement un groupe methyle, ethyle, isopropyle, cyclopropyle, $CF_3$, $CONH_2$, $CO_2CH_3$ $CO_2CH_2CH_3$, CN, $NO_2$, $SCH_3$, $SCH_2CH_3$, un atome d'hydrogène, un halogène, $OCF_3$, $OCH_3$, $OCH_2CH_3$ ou $OCH(CH_3)_2$.

Le groupe (B) des composés de formule (I) dont les substituant X et Y sont définis ci-dessus ou dans le groupe préféré (A) et tels que le groupe $R_1$ représente un halogène, un groupe ethyle, isopropyle, trifluorométhyle, nitrile, nitro, methoxy, ethoxy, isopropoxy, thiométhyle, thioéthyle ou thio iso-propyle, est un groupe de composés préférés et plus particulièrement tels que $R_1$ représente un halogène, un groupe methoxy, ethoxy, thiométhyle, thioéthyle ou trifluorométhyle.

Les composés ci-dessous, ainsi que leurs sels pharmaceutiquement acceptables, solvates et hydrates et leurs conformères ou rotamères sont particulièrement préférés :

2-[(6-Methoxy-pyridin-2-ylamino)-methyl]-phenol
2-[(6-Bromo-pyridin-2-ylamino)-methyl]-phenol
2-[(6-Bromo-pyridin-2-ylamino)-methyl]-4-fluoro-phenol
6-(2-Hydroxy-benzylamino)-pyridine-2-carbonitrile
2-[1-(6-Methoxy-pyridin-2-ylamino)-ethyl]-phenol
2-[(6-Trifluoromethyl-pyridin-2-ylamino)-methyl]-phenol
2-[(6-Chloro-pyridin-2-ylamino)-methyl]-phenol
2-[(6-Ethyl-pyridin-2-ylamino)-methyl]-phenol
2-[(6-Ethoxy-pyridin-2-ylamino)-methyl]-phenol
2-[(6-Isopropoxy-pyridin-2-ylamino)-methyl]-phenol
5-Chloro-2-[(6-methoxy-pyridin-2-ylamino)-methyl]-phenol
2-[(2-Trifluoromethyl-pyrimidin-4-ylamino)-methyl]-phenol
2-[(6-Bromo-pyrazin-2-ylamino)-methyl]-phenol
2-[(2-Chloro-pyrimidin-4-ylamino)-methyl]-phenol
2-[(2-Bromo-pyrimidin-4-ylamino)-methyl]-phenol
2-[(2-Chloro-6-methyl-pyrimidin-4-ylamino)-methyl]-phenol
2-[(6-Chloro-4-trifluoromethyl-pyridin-2-ylamino)-methyl]-phenol
2-[(6-Chloro-4-methyl-pyridin-2-ylamino)-methyl]-phenol
2-[(6-Methoxy-pyrazin-2-ylamino)-methyl]-phenol
2-[(2-Methoxy-pyrimidin-4-ylamino)-methyl]-phenol
2-[(2-Methoxy-6-methyl-pyrimidin-4-ylamino)-methyl]-phenol
2-[(6-Methylsulfanyl-pyridin-2-ylamino)-methyl]-phenol
2-[(6-Methanesulfinyl-pyridin-2-ylamino)-methyl]-phenol
2-[(6-Methanesulfonyl-pyridin-2-ylamino)-methyl]-phenol
2-[(6-Methoxy-pyridin-2-ylamino)-methyl]-6-methyl-phenol
2-[(4-Bromo-6-methoxy-pyridin-2-ylamino)-methyl]-phenol
2-[(6-Bromo-2-methoxy-pyrimidin-4-ylamino)-methyl]-phenol
2-[(4-Chloro-6-methoxy-pyridin-2-ylamino)-methyl]-phenol
2-[(6-Bromo-2-methoxy-pyrimidin-4-ylamino)-methyl]-phenol
2-[(4-Bromo-6-methoxy-pyridin-2-ylamino)-methyl]-6-fluoro-phenol
2-[(4-Bromo-6-methoxy-pyridin-2-ylamino)-methyl]-5-fluoro-phenol
2-[(4-Bromo-6-methoxy-pyridin-2-ylamino)-methyl]-3-fluoro-phenol
2-[(4-Bromo-6-methoxy-pyridin-2-ylamino)-methyl]-4-fluoro-phenol
2-[(6-Bromo-2-methoxy-pyrimidin-4-ylamino)-methyl]-4-fluoro-phenol
2-[(4-Chloro-6-methoxy-pyridin-2-ylamino)-methyl]-4-fluoro-phenol

2-[(6-Chloro-2-methoxy-pyrimidin-4-ylamino)-methyl]-4-fluoro-phenol
2-[1-(4-Bromo-6-methoxy-pyridin-2-ylamino)-ethyl]-phenol
2-[1-(4-Bromo-6-methoxy-pyridin-2-ylamino)-propyl]-phenol
2-[1-(6-Bromo-4-methyl-pyridin-2-ylamino)-1-methyl-ethyl]-phenol
2-[1-(4-Bromo-6-methoxy-pyridin-2-ylamino)-propyl]-4-fluoro-phenol
2-[1-(6-Bromo-pyridin-2-ylamino)-propyl]-4-fluoro-phenol
4-Fluoro-2-[(6-methoxy-pyridin-2-ylamino)-methyl]-phenol
4-Fluoro-2-[1-(6-methoxy-pyridin-2-ylamino)-ethyl]-phenol
4-Fluoro-2-[1-(6-methoxy-pyridin-2-ylamino)-propyl]-phenol
2-[(6-Bromo-4-methoxy-pyridin-2-ylamino)-methyl]-phenol
2-[(6-Bromo-4-methyl-pyridin-2-ylamino)-methyl]-phenol
2-[(6-Chloro-4-methoxy-pyridin-2-ylamino)-methyl]-phenol

La présente divulgation décrit également un procédé de préparation des composés de formule générale (I). Conformément à l'invention on peut préparer les composés de formule (I) par une des trois méthodes décrites dans le **Schéma 1** ci après et éventuellement compléter par une ou plusieurs réactions telles que décrites dans le **Schéma 2**.

## Schéma 1

### Méthode 1a

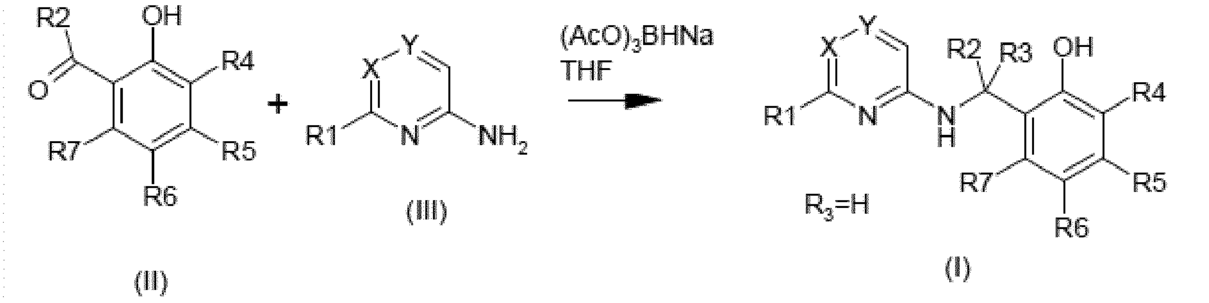


(II) (III) (I)

$R_3$=H

### Méthode 1b

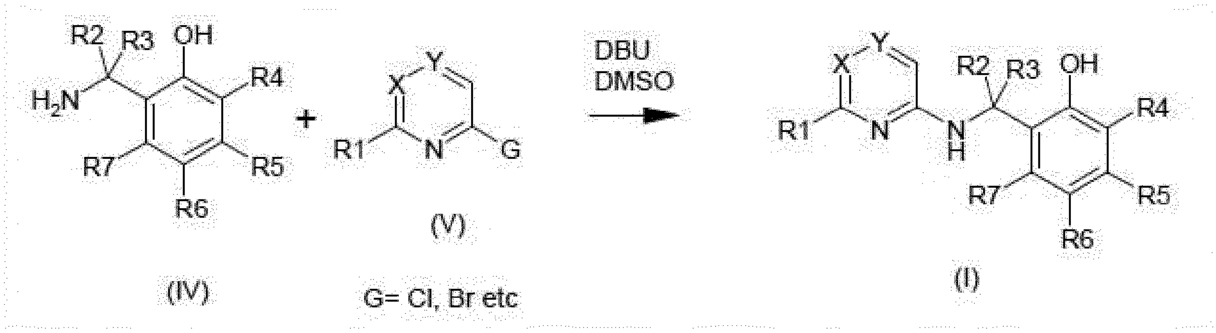


(IV) (V) (I)

G= Cl, Br etc

### Méthode 1c

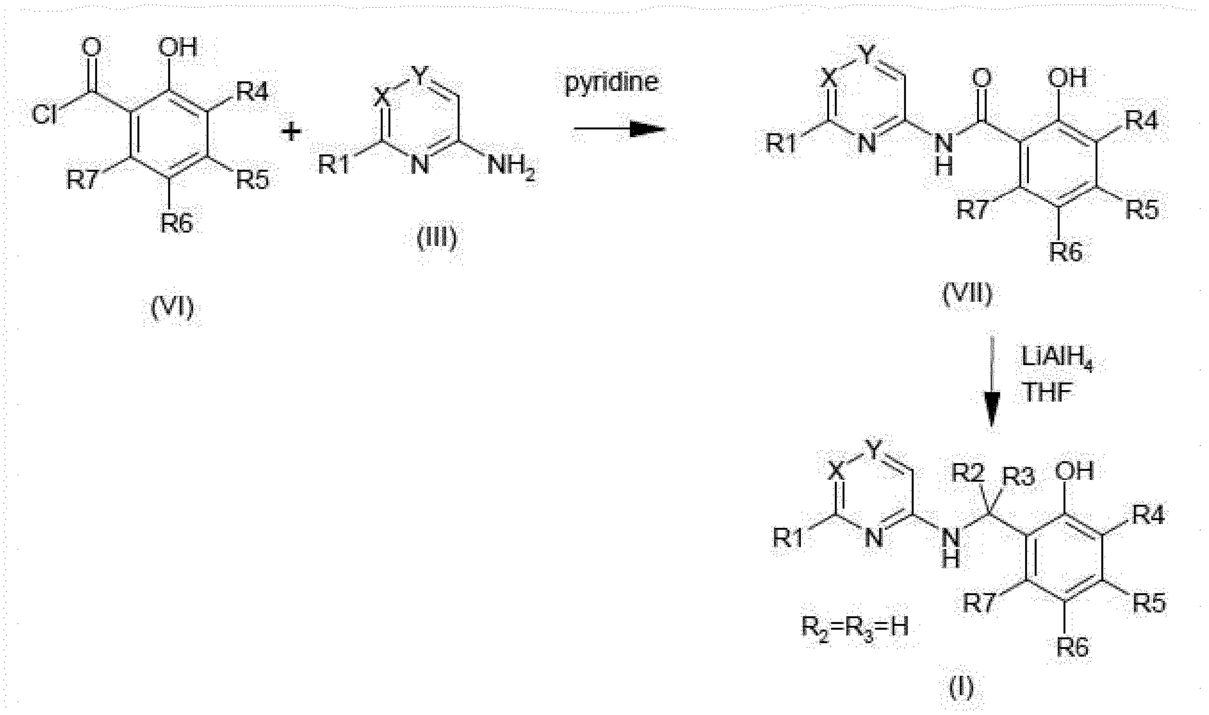


(VI) (III) (VII) (I)

$R_2$=$R_3$=H

Les composés phénoliques de formule (I) dans laquelle $R_1$, $R_2$, $R_3$, $R_4$, $R_5$, $R_6$, $R_7$, X et Y sont tels que définis ci-dessus, peuvent être préparés par réaction d'amination réductrice entre un aldéhyde ou une cétone benzylique (II) et une amine (III) en présence d'un agent réducteur, tel que par exemple et de façon non limitante, le triacétoxyborohydrure

de sodium, selon la **Méthode 1a** illustrée dans le **Schéma 1** et par analogie, par exemple, aux réactions décrites dans Org. Pro. R. & D. (2006) 971 - 1031.

Les composés phénoliques de formule (I) peuvent être préparés par réaction entre des hétérocycles (V) comportant un groupe partant et des amines benzyliques en présence d'une base telle que, de façon non limitante, 1, 8-diazabicyclo [5.4.0] undec-7-ene, par exemple dans un solvent tel que le diméthylsulfoxyde et tel que décrit par la **Méthode 1b** du **Schéma 1**. Par groupe partant on désigne un groupe bien connu de l'homme de l'art tel que, de façon non limitante, un halogène, un mésylate, un tosylate ou un triflate.

La troisième méthode de préparation des composés phénoliques de formule (I) consiste à réduire des intermédiaires amides (VII) par un réactif donneur d'hydrures tel que, de façon non limitante, de l'hydrure de lithium et d'aluminium comme illustré par la **Méthode 1c** du **Schéma 1**. Ces intermédiaires amides peuvent être préparés par réaction entre par exemple et de façon non limitante, un chlorure d'acyle (VI) et une amine (III) dans la pyridine. Les chlorures d'acyles (VI) sont préparés à partir des acides selon des techniques bien connues de l'homme du métier par exemple à reflux dans du chlorure de thionyle.

Certains composés comportant un groupe sulphoxy (X) ou sulphone (XI) peuvent éventuellement être préparés par oxydation de l'intermédiaire thio éther (IX) tel que décrit dans le **Schéma 2** selon la **Méthode 2a**. L'oxydation peut par exemple, et de façon non limitante, être effectuée par l'oxone. L'intermédiaire thio éther (IX) peut être préparé à partir de composés (VIII) comportant un groupe partant tel que, de façon non limitante, un atome de chlore, par réaction avec un thiolate dans le diméthylsulfoxyde. Certains composés comportant un groupe éther peuvent éventuellement être préparés par réaction de l'intermédiaire (VIII) avec l'alcool correspondant tel que par exemple, de façon non limitante, le méthanol en présence d'une base comme l'hydroxyde de sodium éventuellement par chauffage au four microonde et tel que décrit dans le **Schéma 2** selon la **Méthode 2b**.

## Schéma 2

### Méthode 2a

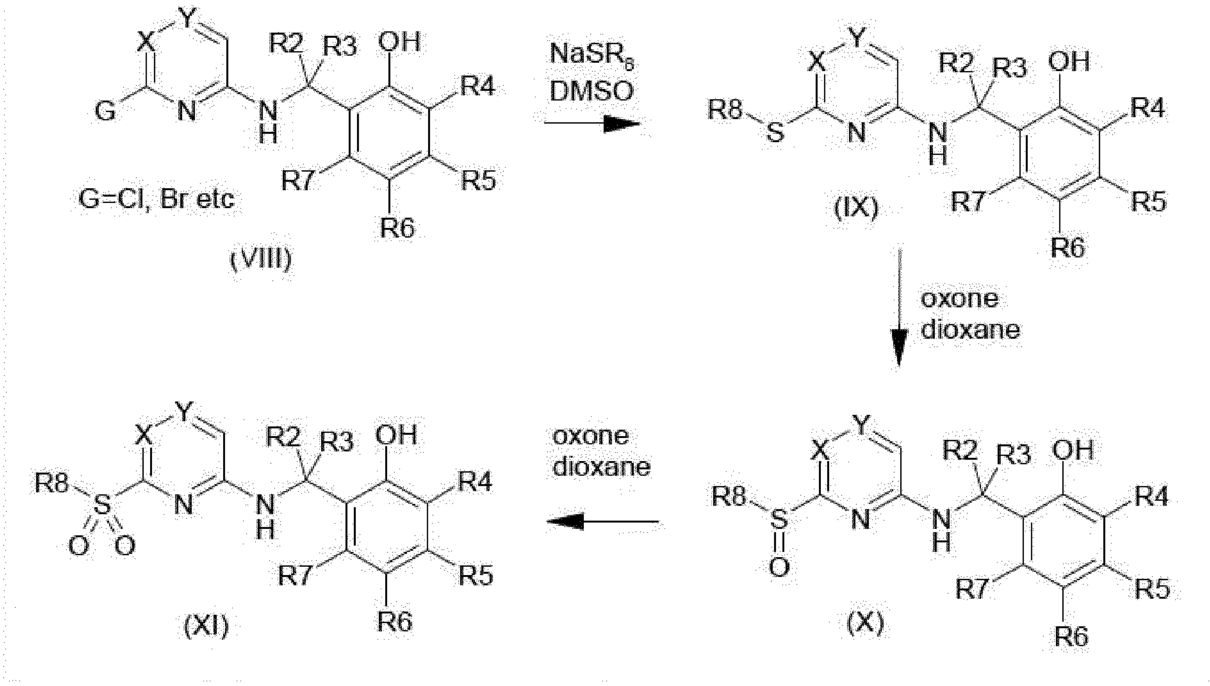


### Méthode 2b

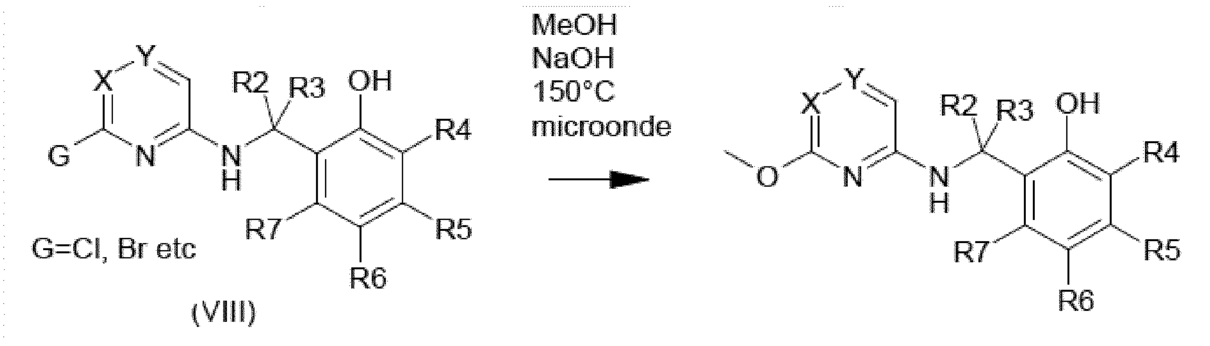


Les groupes fonctionnels éventuellement présents dans les intermédiaires réactionnels utilisés dans le procédé peuvent être protégés, soit de façon permanente, soit de façon temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, des alcools, ou des acides carboxyliques on entend les groupes protecteurs tels que ceux décrits dans « Protective Groups in Organic Chemistry », ed McOmie J. W. F., Plenum Press, 1973, dans « Protective Groups in Organic Synthesis », 2nde édition, Greene T.W. et Wuts P.G.M., ed John Wiley et Sons, 1991 et dans « Protecting Groups », Kocienski P.J., 1994, Georg Thieme Verlag.

Les produits objets de la présente invention sont doués de propriétés pharmacologiques intéressantes; on a constaté notamment qu'ils modulaient l'activité du récepteur aux androgènes.

Des tests donnés dans la partie expérimentale illustrent cette activité modulatrice du récepteur aux androgènes. Les produits objets de la présente invention présentent des activités d'antagonisme ou d'agonisme partiel ou total. Du fait de cette activité, les produits de l'invention peuvent être utilisés comme médicaments chez l'homme ou l'animal.

Ces propriétés rendent les produits de formule générale (I) de la présente invention utilisables comme médicaments pour le traitement des cancers hormonaux dépendant tels que le cancer de la prostate ou le cancer du sein, ainsi que pour lutter contre l'hyperplasie bénigne de la prostate, la puberté précoce, la virilisation, le syndrome des ovaires polykystiques, syndrome de Stein-Leventhal, la perte de libido, l'endométriose. Les composés présentant une activité d'agonisme partiel ou total peuvent en particulier être utilisés pour traiter les afflictions telles que la perte de masse musculaire (sarcopénie), l'atrophie musculaire, l'impuissance et la stérilité masculine, la différentiation masculine anormale (hermaphrodisme), l'hypogonadisme, l'ostéoporose.
Les produits de formule générale (I) de l'invention trouvent leur utilisation cosmétique d'un composé pour l'hygiène corporelle ou capillaire.

Les produits de formule générale (I) de l'invention trouvent également leur utilisation dans le traitement de l'hirsutisme, de l'acné, de la séborrhée, la peau grasse de l'alopécie andro génique, de l'hyperpilosité ou hirsutisme, et ils peuvent être utilisés pour la fabrication d'un médicament pour prévenir et/ou traiter l'hirsutisme, l'alopécie androgénique, de l'hyperpilosité, les désordres de la glande sébacée tels que l'hyperséborrhée, l'acné, la peau grasse ou la dermite séborrhéique. Les produits de formule (I) peuvent donc être utilisés en dermatologie: ils peuvent être utilisés seuls ou en association. Ils peuvent être associés notamment avec un produit antibiotique tels que les dérivés de l'acide azélaique, fusidique, l'érythromycine ou avec un dérivé des rétinoïdes tel que la tretinoïne pour le traitement de l'acné, ou avec un inhibiteur de la 5 a- réductase tel que le (5alpha,17beta)-N-1,1-diméthyléthyl 3-oxo 4-aza-androst-1-ène 17-carboxamide (ou Finastéride Merck, 13ème édition) ou l'acide azélaïque ou un agent bloquant des récepteurs androgènes pour le traitement de l'acné, de l'alopécie ou de l'hirsutisme, ou avec un produit stimulant la croissance des cheveux tel que le Minoxidil pour le traitement de l'alopécie.

La présente invention a également pour objet à titre de médicament les composés de formule (I) tels que décrits ci-dessus, ainsi que leurs sels pharmaceutiquement acceptables, solvates pharmaceutiquement acceptables et/ou hydrates.

A titre d'illustration, plusieurs exemples de préparation de composés actifs de formule (I) selon l'invention, sont donnés ci-après, ainsi que des résultats d'activité biologiques de tels composés.

**MODES OPERATOIRES**

**Exemple 1 : 2-[(6-Methoxy-pyridin-2-ylamino)-methyl]-phenol**

Synthèse selon le Schéma 1, Méthode 1a

512 mg (2.41 mmol, 1.5 eq) de triacetoxyborohydrure de sodium sont ajoutés à une solution de 200 mg (1.61 mmol, 1 eq) de 6-methoxy-pyridin-2-ylamine (**produit de départ 1**), 236 mg (2.41 mmol, 1 eq) de 2-hydroxy-benzaldehyde (**produit de départ 2**) dans 20 ml de tétrahydrofuranne. La solution est agitée à température ambiante pendant 48h. Elle est évaporée et le résidu est repris par 100 ml de dichlorométhane puis extrait avec une solution aqueuse saturée en chlorure d'ammonium. La phase aqueuse est extraite 2 fois avec du dichlorométhane. Les phases organiques sont rassemblées et sont séchées sur sulfate de sodium. Le résidu est chromatographié sur gel de silice (acétate d'éthyle / heptane 5/95). 2-[(6-methoxy-pyridin-2-ylamino)-methyl]-phenol est obtenu sous la forme d'un solide blanc. Point de fusion = 103°C.
RMN 1H (CDCl3): 3.94 (s, 3H); 4.52 (d, 2H, J= 3.08 Hz); 4.95 (s, 1H), 6.03 (dd, 2H, J= 6.2 Hz, J'= 1.64 Hz); 6.85 (t, 1H, J= 6.28 Hz, J'= 7.4 Hz); 6.95 (d, 1H, J= 9.04 Hz); 7.15- 7.23 (m, 2H); 7.36 (t, 1H, J= 7.92 Hz, J'= 7.96 Hz); 10.21 (s, 1H)

Préparation de l'intermédiaire 6-Amino-pyridine-2-carbonitrile

340 mg (2.89 mmol, 1 eq) de cyanure de zinc sont ajoutés à 500 mg (2.89 mmol, 1 eq) de 6-bromo-pyridin-2-ylamine dans 10 ml de diméthylformamide dans un tube de micro-ondes. 170 mg (0.147 mmol, 0.05 eq) de tetrakis(triphenylphosphine)palladium sont ajoutés. Le milieu est chauffé à 170°C pendant 1 heure 30 au four micro-onde. 50 ml d'acétate d'éthyle sont rajoutés au milieu qui est filtré sur célite. Le filtrat est lavé à l'eau et extrait avec de l'acétate d'éthyle. Les phases organiques sont rassemblées et sont séchées sur sulfate de sodium. Le résidu est trituré dans de l'heptane. 6-Amino-pyridine-2-carbonitrile est obtenu sous la forme d'un solide orangé.
Point de fusion = 92°C.

Préparation de l'intermédiaire 6-Ethoxy-pyridin-2-ylamine

Dans un tube micro-onde, on introduit 500mg (2.89mmoles) de 2 amino 6 bromopyridine, auquel on ajoute 2ml d'éthanol, et 231 mg (5.78mmoles, 2eq) d'hydroxyde de sodium. Le mélange est chauffé pendant 10 heures au four à micro-onde à 170°C. Le milieu réactionnel est dilué avec 50ml de dichlorométhane puis lavé avec deux fois 50ml d'eau. La phase organique est concentrée à sec et le résidu est purifié par chromatographie sur silice avec comme éluant heptane/acétate d'éthyle (1/1). On obtient le produit attendu sous la forme d'une huile incolore.

**Préparation de l'intermédiaire 6-Isopropoxy-pyridin-2-ylamine**

On prépare cet intermédiaire selon le mode opératoire décrit pour 6-ethoxy-pyridin-2-ylamine en remplaçant l'éthanol par de l'isopropanol. On obtient le produit attendu sous la forme d'une huile incolore.

**Exemple 2 à 12**

Les exemples 2 à 12 sont décrits dans le tableau 1 ci-dessous. Les composés sont synthétisés suivant le mode opératoire ci dessus, en remplaçant les **produits de départs 1 et 2** évoqués dans l'exemple 1 par les produits mentionnés dans le tableau 1.

**Tableau 1**

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet, J= constante de couplage en Hz) |
|---|---|---|---|---|---|
| 2 | 2-[(6-bromo-pyridin-2-ylamino)-methyl]-phenol | 2-Bromo-pyridin-6-ylamine | 2-Hydroxy-benzaldehyde | 127 | (CDCl3) 4.48 (d, 2H, J= 4.28 Hz); 5.17 (s, 1H); 6.35 (d, 1H, J= 8.7 Hz); 6.77 (d, 1H, J= 7.3 Hz); 6.87 (t, 1H, J= 7.4 Hz); 7.0 (d, 1H, J= 9.1 Hz); 7.16- 7.27 (m, 3H); 9.84 (s, 1H) |
| 3 | 2-[(6-bromo-pyridin-2-ylamino)-methyl]-4-fluoro-phenol | 6-Bromo-pyridin-2-ylamine | 5-Fluoro-2-hydroxy-benzaldehyde | 143 | (DMSO) 4.32 (d, 2H, J= 6 Hz); 6.5 (d, 1H, J= 8.2 Hz); 6.65 (d, 1H, J= 7.4 Hz); 6.77- 6.81 (m, 1H); 6.85-6.95 (m, 2H); 7.3 (t, 1H, J= 8.2 Hz); 7.32-7.35 (m, 1H); 9.59 (s, 1H) |
| 4 | 6-(2-hydroxy-benzylamino)-pyridine-2-carbonitrile | 6-Amino-pyridine-2-carbonitrile | 2-Hydroxy-benzaldehyde | 153 | (DMSO) 4.36 (d, 2H, J= 4.8 Hz); 6.73 (t, 1H, J= 7.4 Hz); 6.81-6.84 (m, 2H); 7.04-7.08 (m, 2H); 7.14 (d, 1H, J= 7.4 Hz); 7.46-7.54 (m, 2H); 9.60 (s, 1H) |

(suite)

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet, J= constante de couplage en Hz) |
|---|---|---|---|---|---|
| 5 | 2-[1-(6-methoxy-pyridin-2-ylamino)- ethyl]-phenol | 6-Methoxy-pyridin-2-ylamine | 1-(2-Hydroxy-phenyl)-ethanone | 109 | (DMSO) 1.35 (d, 3H, J= 6.8 Hz); 3.66 (s, 3H); 5.13-5.16 (m, 1H); 5.79 (d, 1H, J= 7.7 Hz); 5.94 (d, 1 H, J= 7.8 Hz); 6.7 (t, 1H, J= 7.3 Hz); 6.77 (t, 2H, J= 8.2 Hz); 6.97 (t, 1H, J= 7.6 Hz); 7.20- 7.24 (m, 2H); 9.42 (s, 1H) |
| 6 | 2-[(6-trifluoromethyl-pyridin-2-ylamino)-methyl]-phenol | 2-amino-6-(trifluoromethyl) pyridine | 2-Hydroxy-benzaldehyde | 125 | (DMSO) 4.38 (d, 2H, J=5.4Hz); 6.71-6.82 (m, 3H); 6.88 (d, 1H, J=7.2Hz); 7.06 (t, 1H, J=7.6Hz); 7.19 (d, 1H, J=7.4Hz); 7.37-7.40 (m, 1H); 7.56 (t, 1H, J=7.8Hz); 9.56 (s, 1H) |
| 7 | 2-[(6-chloropyridin-2-ylamino)-methyl]-phenol | 2-amino-6-chloropyridine | 2-Hydroxy-benzaldehyde | Non déterminé | (DMSO) 4.34 (d, 1H, J=5.8Hz); 6.45-6.50 (m, 2H); 6.72-6.76 (m, 2H) 6.81 (d, 1H, J=8Hz); 7.06 (t, 1H, J=7.6Hz); 7.15 (d, 1H, J=7.4Hz); 7.26-7.29 (m, 1H); 7.38 (t, 1H, J=7.5); 9.57 (s, 1H) |

(suite)

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet, J= constante de couplage en Hz) |
|---|---|---|---|---|---|
| 8 | 2-[(6-ethyl-pyridin-2-ylamino)-methyl]-phenol | 6-Ethyl-pyridin-2-ylamine | 2-Hydroxy-benzaldehyde | Non déterminé | (DMSO) 1.2 (t, 3H, J=7.6Hz); 2.54-2.60 (q, 2H, J=7.5Hz); 4.31 (d, 2H, J=6.12Hz); 6.33-6.38 (m, 2H); 6.72-6.78 (m, 2H); 7.06-7.12 (m, 2H); 7.18 (d, 1H, J=7.4Hz); 7.3 (t, 1H, J=7.5Hz); 10.88 (s, 1H) |
| 9 | 2-[(6-ethoxy-pyridin-2-ylamino)-methyl]-phenol | 2-amino-6-ethoxypyridine | 2-Hydroxy-benzaldehyde | 87 | (CD3OD) 1.35 (t, 3H, J=7.0Hz); 3.31-3.33 (q, 2H, J=7.0Hz); 4.42 (s, 2H); 5.91-5.93 (m, 1H); 6.05 (d, 1H, J=7.8Hz); 6.75-6.80 (m, 2H); 7.05-7.10 (m, 1H); 7.19-7.22 (m, 1H); 7.29-7.33 (m, 1H) |
| 10 | 2-[(6-isopropoxypyridin-2-ylamino)-methyl]-phenol | 2-amino-6 isopropoxypyridine | 2-Hydroxy-benzaldehyde | Non déterminé | (CD3OD) 1.26 (t, 6H, J=6.1Hz); 4.43 (s, 2H); 5.03-5.09 (m, 1H); 5.88-5.90 (m, 1H); 6.02-6.04 (m, 1H); 6.74-6.79 (m, 2H); 7.04-7.08 (m, 1H); 7.19-7.21 (m, 1H); 7.3 (t, 1H, J=7.9Hz) |

(suite)

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet, J= constante de couplage en Hz) |
|---|---|---|---|---|---|
| 11 | 5-chloro-2-[(6-methoxy-pyridin-2-ylamino)-methyl]-phenol | 6-Methoxy-pyridin-2-ylamine | 4-Chloro-2-hydroxy-benzaldehyde | Non déterminé | (DMSO) 3.72 (s, 3H) ; 4.39 (d, 2H, J=5.9Hz) ; 5.86 (d, 1H, J=7.8Hz); 6.03 (d, 1H, J=7.9Hz); 6.7 (m, 1H); 6.8 (s, 1H); 6.93 (m, 1H); 7.21 (d, 1H, J=8.4Hz); 7.28 (t, 1H, J=7.8Hz); 9.73 (s, 1H) |
| 12 | 2-[(2-trifluoromethyl-pyrimidin-4-ylamino)-methyl]-phenol | 2-Trifluoromethyl-pyrimidin-4-ylamine | 2-Hydroxy-benzaldehyde | 201 | (DMSO) 4.34-4.47 (m, 2H); 6.55-6.85 (m, 3H); 7.1 (t, 1H, J=7.4Hz); 7.19 (d, 1H, J=7.3Hz); 8.15 (d, 1H, J=5.9Hz); 8.39-8.47 (m, 1H); 9.63 (s, 1H) |

**Exemple 13 : 2-[(6-Bromo-pyrazin-2-ylamino)-methyl]-phenol**

Synthèse selon le Schéma 1, Méthode 1b

Dans un ballon de 50mL on introduit 1g (4.2mmoles) de 2,6-dibromo-pyrazine (**produit de départ 3**), auquel on ajoute 15ml de diméthylsulfoxyde, 638mg (4.2mmoles, 1 eq) de 1, 8-Diazabicyclo [5.4.0] undec-7-ene, et 1.03g (8.4mmoles, 2eq) de 2-hydroxybenzylamine (**produit de départ 4**), on laisse agiter pendant 2H à température ambiante. Le milieu réactionnel est dilué avec 50ml d'acétate d'éthyle, on lave avec 50ml d'une solution de chlorure d'ammonium saturée puis avec deux fois 50ml d'eau. Les phases organiques sont séchées sur sulfate de magnesium, filtrées et concentrées à sec.

Le résidu est purifié par chromatographie sur silice avec comme éluant un mélange heptane/acétate d'éthyle (8/2). 2-[(6-Bromo-pyrazin-2-ylamino)-methyl]-phenol est obtenu sous la forme d'un solide blanc.
Point de fusion = 168°C.
RMN 1H (DMSO): 4.36 (d, 1H, J=5.3Hz); 6.74-6.76 (m, 1H); 6.83 (dd, 1H); 7.07-7.11 (m, 1H); 7.17 (dd, 1H); 7.75 (s, 1H); 7.80-7.83 (m, 1H); 7.96 (s, 1H); 9.61 (s, 1H)

**Exemple 14 à 18**

Les exemples 14 à 18 sont décrits dans le tableau 2 ci-dessous. Les composés sont synthétisés suivant le mode opératoire ci dessus, en remplaçant les **produits de départs 3 et 4** évoqués dans l'exemple 13 par les produits mentionnés dans le tableau 2.

**Tableau 2**

| Exemple # | Nom IUPAC | Produit de départ 3 | Produit de départ 4 | Point de fusion (°C) | RMN 1H-400Mhz - (s= singulet, d= doublet, t=triplet, m=multiplet, q=quadruplet, J= constante de couplage en Hz) |
|---|---|---|---|---|---|
| 14 | 2-[(2-chloro-pyrimidin-4-ylamino)-methyl]-phenol | 2,4 dichloro-pyrimidine | 2-hydroxybenzylamine | Non déterminé | (CD3OD) 4.53 (m, 2H); 6.45 (d, 1H, J=6.0Hz); 6.78-6.83 (m, 2H); 7.10-7.14 (m, 1H); 7.22 (d, 1H, J=6.9Hz); 7.83 (m, 1H) |
| 15 | 2-[(2-bromo-pyrimidin-4-ylamino)-methyl]- phenol | 2,4 dibromo-pyrimidine | 2-hydroxybenzylamine | Non Déterminé | (CD3OD) 4.42-4.52 (m, 2H) 6.48 (d, 1H, J=6.0Hz); 6.78-6.83 (m, 2H); 7.12 (t, 1H, J=8.2Hz); 7.21-7.23 (m, 1H); 7.76-7.86 (m, 1H) |
| 16 | 2-[(2-bhloro-6-methyl-pyrimidin-4-ylamino)-methyl]- phenol | 2,4 dichloro-6-méthyl pyrimidine | 2-hydroxybenzylamine | Non Déterminé | (DMSO) 2.17 (s, 3H); 4.40 (s, 2H); 6.35 (s, 1H);6.74(t, 1H, J=7.4Hz); 6.82 (d, 1H, J=7.9Hz); 7.06-7.12 (m, 2H); 8.04 (s, 1H); 9.60 (s, 1H). |
| 17 | 2-[(6-chloro-4-trifluoromethyl-pyridin-2-ylamino)-methyl]-phenol | 2,6-Dichloro-4-trifluoromethyl-pyridine | 2-hydroxybenzylamine | Non Déterminé | (DMSO) 4.39 (d, 2H, *J*=5.4Hz); 6.73-6.84 (m, 4H); 7.080 (t, 1H, *J*=7.6Hz); 7.17 (d, 1H, *J*=7.4Hz); 7.83 (s, 1H); 9.61 (s, 1H) |

(suite)

| Exemple # | Nom IUPAC | Produit de départ 3 | Produit de départ 4 | Point de fusion (°C) | RMN 1H-400Mhz - (s= singulet, d= doublet, t=triplet, m=multiplet, q=quadruplet, J= constante de couplage en Hz) |
|---|---|---|---|---|---|
| 18 | 2-[(6-chloro-4-methyl-pyridin-2-ylamino)-methyl]-phenol | 2,6-Dichloro-4-methyl- pyridine | 2-hydroxybenzylamine | 138 | (DMSO) 2.12 (s, 3H); 4.32 (d, 2H, *J*=5.5Hz); 6.28 (s, 1H); 6.37 (s, 1H); 6.73 (t, 1H, *J*=7.4Hz); 6.8 (d, 1H, *J*=8Hz); 7.04 (t, 1H, 7.7Hz); 7.12-7.17 (m, 2H); 9.58 (s, 1H) |

**Exemple 19 : 2-[(6-Methoxy-pyrazin-2-ylamino)-methyl]-phenol**

Synthèse selon le Schéma 2, Méthode 2b

Dans un tube micro-onde, on introduit 363mg (1.29mmoles) de 2-[(6-bromo-pyrazin-2-ylamino)-methyl]-phenol, préparé comme décrit précedemment dans l'exemple 12, auquel on ajoute 3ml de méthanol et 103mg (2.58mmoles, 2eq) d'hydroxyde de sodium. Le milieu réactionnel est alors chauffé pendant 30 min au four micro-onde à 150°C puis est dilué avec 50ml d'acétate d'éthyle. On neutralise avec une solution de chlorure d'ammonium jusqu'à pH =7, on décante, on lave la phase organique avec deux fois 50ml d'eau. La phase organique est séchée sur sulfate de magnésium, filtrée et concentrée à sec. Le résidu est purifié par chromatographie sur silice avec comme éluant heptane/acétate d'éthyle (7/3). 2-[(6-Methoxy-pyrazin-2-ylamino)-methyl]-phenol est obtenu sous forme d'un solide blanc
Point de fusion = 158°C.
RMN 1H (DMSO) 3.78 (s, 1H); 4.40 (d, 2H, J=5.2Hz); 6.73 (t, 1H, J=7.4Hz); 6.81 (d, 1H, J=8Hz); 7.05 (t, 1H, J=7.8Hz); 7.19 (d, 1H, J=7.4); 7.26 (s, 1H); 7.31-7.32 (m, 1H); 7.50 (s, 1H); 9.55 (s, 1H).

**Exemple 20 : 2-[(2-Methoxy-pyrimidin-4-ylamino)-methyl]-phenol**

On prépare ce composé selon le mode opératoire décrit pour l'exemple 19 à partir de 2-[(2-chloro-pyrimidin-4-ylamino)-methyl]-phenol. 2-[(2-Methoxy-pyrimidin-4-ylamino)-methyl]-phenol est obtenu sous la forme d'un solide blanc.
Point de fusion = 161°C.
RMN 1H (CD3OD) 3.90 (s, 3H); 4.53 (s,3H); 6.15 (d, 2H, J=6.0Hz);6.77-6.81 (m, 2H); 7.07-7.12 (m,1H); 7.21 (d, 1H, J=7.4Hz); 7.78 (s, 1H)

**Exemple 21 : 2-[(2-Methoxy-6-methyl-pyrimidin-4-ylamino)-methyl]-phenol**

On prépare ce composé selon le mode opératoire décrit pour l'exemple 19 ci-dessus à partir de 2-[(2-Chloro-6-methyl-pyrimidin-4-ylamino)-methyl]-phenol.
RMN 1H (DMSO) 2.12 (s, 3H); 3.74 (s, 3H); 4.38 (m, 2H); 6.04 (s, 1H); 6.73 (t, 1H, J=7.4Hz); 6.80 (d, 1H, J=8.0Hz); 7.06(t, 1H, J=7.7Hz); 7.11 (d, 1H, J=7.3Hz); 7.65 (s, 1H); 9.71 (s, 1H).

**Exemple 22 : 2-[(6-Methylsulfanyl-pyridin-2-ylamino)-methyl]-phenol**

Synthèse selon le Schéma 2, Méthode 2a

Dans un tube micro-onde, on introduit 300mg (1.28mmoles) de 2-[(6-chloro-pyridin-2-ylamino)-methyl]-phenol, auquel on ajoute 5ml de diméthylsulfoxyde et 448mg (6.4mmoles, 5eq) de methane-thiolate de sodium. On chauffe pendant 16h à 90°C. Le milieu réactionnel est dilué avec 50ml d'acétate d'éthyle puis lavé avec 50ml d'une solution de chlorure d'ammonium saturée puis deux fois 50ml d'eau distillé. La phase organique est séchée sur sulfate de magnésium puis filtrée et concentrée à sec. Le résidu est purifié par chromatographie sur 40g de silice avec comme éluant heptane/acétate d'éthyle (7/3). Le produit obtenu est remis en solution dans de l'acétate d'éthyle, on ajoute de l'heptane jusqu'à apparition d'un trouble, on refroidit à 0°C, on filtre. On obtient le 2-[(6-methylsulfanyl-pyridin-2-ylamino)-methyl]-phenol sous forme d'un solide blanc.
Point de fusion = 61°C.
RMN 1H (DMSO) 2.38 (s, 3H); 4.38 (d, 2H, J=5.6Hz); 6.21 (d, 1H, J=8.2Hz); 6.34 (d, 1H, J=7.4Hz); 6.72 (t, 1H, 7.3Hz); 6.93-6.96 (m, 1H); 7.04 (t, 1H, J=7.7Hz); 7.15 (d, 1H, J=7.1Hz); 7.23 (t, 1H, J=7.6Hz); 9.65 (s, 1H).

**Exemple 23 : 2-[(6-Methanesulfinyl-pyridin-2-ylamino)-methyl]-phenol**

On mélange 160mg (0.66mmoles) de 2-[(6-methanesylfanyl-pyridin-2-ylamino)-methyl]-phenol et 406mg (0.66mmoles, 1 eq) d'oxone dans 20ml de dioxane. Après une heure d'agitation à température ambiante, le milieu réactionnel est chauffé à 90°C pendant 4h. Après retour à température ambiante, le milieu réactionnel est dilué avec 50ml d'acétate d'éthyle puis lavé par deux fois 50ml d'eau. La phase organique est séchée sur sulphate de magnésium, filtrée et concentrée à sec. Le résidu est purifié par chromatographie sur silice en éluant avec un mélange heptane/acétate d'éthyle (1/1). 2-[(6-Methanesulfinyl-pyridin-2-ylamino)-methyl]-phenol est obtenu sous forme d'un solide blanc.
Point de fusion = 133°C.
RMN 1H (CDCl3) 2.89 (s, 3H); 4.51 (d, 2H, J=6.2Hz); 5.32-5.33 (m, 1H); 6.5 (dd, 1H); 6.87-6.95 (m, 2H); 7.19-7.28 (m, 2H); 7.30-7.59 (m, 1H); 7.62 (t, 1H, J=7.3Hz); 9.28 (s, 1H).

**Exemple 24 : 2-[(6-Methanesulfonyl-pyridin-2-ylamino)-methyl]-phenol**

On mélange 80mg (0.33mmoles) de 2-[(6-methanesylfanyl-pyridin-2-ylamino)-methyl]-phenol et 406mg (0.66mmoles, 2eq) d'oxone dans 20ml de dioxane et on chauffe pendant 16h à 90°C. Le milieu réactionnel est dilué avec 50ml d'acétate d'éthyle puis lavé avec deux fois 50ml d'eau. La phase organique est séchée sur sulphate de magnésium, filtrée et concentrée à sec. Le résidu est purifié par chromatographie sur silice en éluant avec un mélange heptane/AcOEt (1/1). Le 2-[(6-methanesulfonyl-pyridin-2-ylamino)-methyl]-phenol est obtenu sous forme d'un solide légèrement vert.
RMN1H (CDCl3) 3.12 (s, 3H); 5.32-5.33 (m, 1 H); 6.58 (d, 1H, J=7.9Hz); 6.79-6.83 (m, 1H); 6.87 (d, 1H, J=7.4Hz); 7.3 (d, 1H, J=6.6Hz); 7.5 (t, 1H, J=7.2Hz); 8.56 (s, 1H).

**Exemple 25 : 2-[(6-Methoxy-pyridin-2-ylamino)-methyl]-6-methyl-phenol**

Synthèse selon le Schéma 1, Méthode 1c

80mg (2.1mmoles, 6eq) d'hydrure d'aluminium et de lithium sont ajoutés par petites fractions à un mélange de 90mg (0.35mmoles) de 2-hydroxy-N-(6-methoxy-pyridin-2-yl)-3-methyl-benzamide dans 10ml de dioxane. Le milieu réactionnel est chauffé à 80°C pendant 16h. 80mg (2.1mmoles, 6eq) d'hydrure d'aluminium et de lithium sont ajoutés à nouveau et on chauffe à 80°C pendant 4H. On dilue le milieu réactionnel avec 50ml d'acétate d'éthyle, on lave avec 50ml d'une solution saturée de chlorure d'ammonium puis deux fois 50ml d'eau. La phase organique est sèchée sur sulphate de magnésium, filtrée, et concentrée à sec. Le résidu est purifié par chromatographie sur silice en éluant avec un mélange d'heptane/acétate d'éthyle (1/1). Le 2-[(6-methoxy-pyridin-2-ylamino)-methyl]-6-methyl-phenol est obtenu sous forme d'un solide blanc.
RMN 1H (CDCl3) 2.19 (s, 3H); 3.89 (s, 3H); 4.46 (d, 2H, j=6.7Hz); 4.75 (s, 1H); 5.93-5.97(m, 2H); 6.68 (t, 1H, J=7.4Hz); 6.92 (d, 1H, J=7.5Hz); 7.0 (d, 1H, J=7.4Hz); 7.27 (t, 1H, J=7.9Hz), 9.66 (s, 1H).

Préparation de l'intermédiaire **2-Hydroxy-N-(6-methoxy-pyridin-2-yl)-3-methyl-benzamide**

10ml de chlorure de thionyle sont ajoutés à 1.47g (16.11mmoles) de 2-hydroxy-3-methyl-benzoic acid et le mélange réactionnel est chauffé à 90°C pendant 2h. On concentre à sec le milieu réactionnel en azéotropant avec du

toluène. Puis le résidu est mis en solution dans 10ml de pyridine, auquel on ajoute au goutte à goutte 600mg (4.83mmoles, 1 eq) de 2 méthoxy-pyridin-6-amine, et on laisse agiter à température ambiante pendant 1h30. 30ml de soude 1M (19.34mmoles, 4eq) sont ajoutés et on chauffe à 60°C pendant 16h. Le milieu réactionnel est dilué avec 100ml d'acétate d'éthyle, la phase aqueuse est extraite et lavée avec 50mL d'acétate d'éthyle. La phase aqueuse est ensuite acidifiée à 0°C avec HCl 37% au goutte à goutte jusqu' à pH=4. On extrait les phases organiques avec deux fois 50ml d'acétate d'éthyle puis elles sont lavées avec deux fois 50ml d'eau. On concentre à sec les phases organiques et le résidu est purifié par chromatographie sur silice en éluant avec un mélange d'heptane/acétate d'éthyle (1/1). 2-Hydroxy-N-(6-methoxy-pyridin-2-yl)-3-methyl- benzamide est obtenu sous forme d'un solide blanc.
RMN 1H (CDCl3) 2.23 (s, 3H); 3.84 (s, 3H); 6.48 (d, 1H, *J*=8Hz); 6.78 (t, 1H, *J*=7.7Hz); 7.26 (d, 1H, *J*=7.3Hz); 7.38 (d, 1H, *J*=8Hz); 7.58 (t, 1H, *J*=8Hz); 7.76 (d, 1H, *J*=7.7Hz); 8.31 (s, 1H); 12.12 (s, 1H).

Tous les spectres de RMN (résonance magnétique nucléaire) sont en accord avec les structures proposées. Les déplacements chimiques sont exprimés en partie par million. La référence interne est le tetraméthylsilane. Les abréviations suivantes sont utilisées : CDCl3 = chloroforme deutérié, DMSO= diméthylsulphoxide deutérié, CD3OD = méthanol deutérié.

**Exemple 26 : Tests biologiques**

Les composés selon l'invention présentent des propriétés inhibitrices des récepteurs de type AR. Cette activité inhibitrice des récepteurs AR est mesurée dans un test de transactivation par les constantes de dissociation KdR (repos), KdA (actif) et Kdapp (apparent) d'après la méthode exposée dans J. Motecular Biology (1965), 12(1), 88-118, Monod J. *et al.*
Par inhibiteur des récepteurs de type ARs, on entend selon l'invention tout composé qui présente une constante de dissociation Kdapp inférieure ou égale à 1 μM, et un rapport KdR/ KdA ≤ 10, dans un test de transactivation.
Les composés préférés de la présente invention présentent une constante de dissociation inférieure ou égale à 500 nM, et avantageusement inférieur ou égal à 100 nM.
Le test de transactivation est réalisé dans la lignée cellulaire PALM (PC3 Androgene receptor Luciferase MMTV) qui est un transfectant stable contenant les plasmides PMMTV- neo-Luc (gène reporter) et pSG5puro-AR.
Dans cette étude, l'affinité de chaque produit pour les 2 états des récepteurs (KdR et KdA) est déterminée ainsi qu'un Kd apparent (KdApp). Cette constante est une résultante des 2 Kd mais dépend également de l'équilibre initial du récepteur entre l'état actif et l'état repos ($L_0$) et de son taux d'expression. Sa détermination se fait par la formule suivante :

$$1/KdApp=(L0/(1+L0))x(1/KdR) +(1/(1+L0))x(1/KdA)$$

Pour déterminer ces constantes, des « courbes croisées » du produit à tester contre un agoniste de référence, le methyltrienolone, sont réalisées en plaque 96 puits. Le produit à tester est utilisé à 10 concentrations et l'agoniste de référence à 7 concentrations.

A titre illustratif, un Kdapp de 40 nM est obtenu pour le composé (1), un Kdapp de 2nM est obtenu pour le composé (2), un Kdapp de 8 nM est obtenu pour le composé (19), un Kdapp de 1000 nM est obtenu pour le composé (18), un Kdapp de 200 nM est obtenu pour le composé (4).

## Revendications

1. Composés de formule (I) :

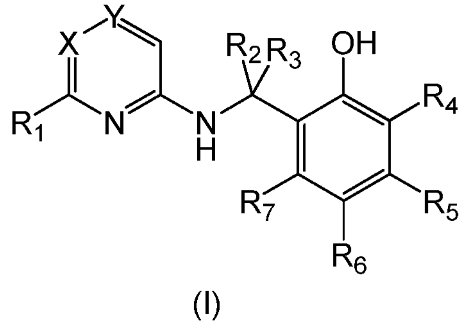

(I)

dans laquelle :

- $R_1$ représente un groupe $C_{2-6}$ alkyle, $C_{3-7}$ cycloalkyle, $C_{1-6}$ alkyloxy, $-S(O)_m-C_{1-6}$ alkyle, $C_{1-6}$ fluoroalkyle, $C_{1-6}$ fluoroalkyloxy, $-(CH_2)_n-C_{3-9}$ cycloalkyle, $C_{2-6}$ alkyle-OH, $-(CH_2)_n-C_{1-6}$ alkyloxy, $-(CH_2)_n-C_{1-6}$ fluoroalkyle, $-(CH_2)_p-O-C_{1-6}$ fluoroalkyle, $COR_a$, CN, $NO_2$, $NR_8R_9$, un halogène, un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents,
- $R_2$ et $R_3$ sont identiques ou différents et représentent un atome d'hydrogène ou un groupe $C_{1-9}$ alkyle, $C_{3-9}$ cycloalkyle, $C_{1-6}$ fluoroalkyle, $-(CH_2)_r-C_{3-9}$ cycloalkyle, $-C_{2-6}$ alkyle-OH, $-(CH_2)_r-C_{1-6}$ alkyloxy, $-(CH_2)_r-C_{3-7}$ cycloalkyle, $-(CH_2)_r-C_{1-6}$ fluoroalkyle,$-(CH_2)_q-O-C_{1-6}$ fluoroalkyle
Eventuellement les groupes $R_2$ et $R_3$ peuvent former avec l'atome de carbone qui les porte un groupe $C_{3-9}$ cycloalkyle ou un hétérocycle tel que tetrahydrofuranne, tetrahydropyranne, tetrahydrothiopyranne, tetrahydro-1 oxy-thiopyranne ou tetrahydro-1,1 dioxy thiopyranne.
- $R_4$, $R_5$, $R_6$, $R_7$ sont identiques ou différents et représentent soit un atome d'hydrogène soit un groupe $C_{1-6}$ alkyle, $C_{3-7}$ cycloalkyle, $C_{1-6}$ alkyloxy, $-S(O)_s-C_{1-6}$ alkyle, $C_{1-6}$ fluoroalkyle, $C_{1-6}$ fluoroalkyloxy, $-(CH_2)_t-C_{3-9}$ cycloalkyle, $-(CH_2)_t-C_{3-9}$ cycloalkyle, $-C_{1-6}$ alkyle -OH, $-(CH_2)_t-C_{1-6}$ alkyloxy, $-(CH_2)_t-C_{1-6}$ fluoroalkyle,$-(CH_2)_u-O-C_{1-6}$ fluoroalkyle, $COR_d$, CN, $NR_8R_9$, ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_c$ identiques ou différents
- X représente CH ou N
- Y représente soit un atome d'azote, soit un atome de carbone substitué par un groupe $C_{1-6}$ alkyle, $C_{3-7}$ cycloalkyle, $C_{1-6}$ alkyloxy, $-S(O)_v-C_{1-6}$ alkyle, $C_{1-6}$ fluoroalkyle, $C_{1-6}$ fluoroalkyloxy, $-(CH_2)_l-C_{3-9}$ cycloalkyle, $-(CH_2)_l-C_{3-9}$ cycloalkyle, $C_{1-6}$ alkyle-OH, $-(CH_2)_l-C_{1-6}$ alkyloxy, $-(CH_2)_l-C_{1-6}$ fluoroalkyle, $-(CH_2)_w-O-C_{1-6}$ fluoroalkyle, $COR_e$, CN, $NR_{10}R_{11}$, $NO_2$, un atome d'hydrogène ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents
- $R_a$, $R_d$ et $R_e$ sont identiques ou différents et représentent un groupe $C_{1-6}$ alkyle, $C_{1-6}$ alkyloxy ou $NR_{12}R_{13}$,
- $R_b$ et $R_c$ sont identiques ou différents et représentent un halogène, un groupe $C_{1-6}$ alkyle, $C_{3-7}$ cycloalkyle, $C_{1-6}$ alkyloxy, $-S(O)_j-C_{1-6}$ alkyle, $C_{1-6}$ fluoroalkyle, $C_{1-6}$ fluoroalkyloxy, $-(CH_2)_i-C_{3-7}$ cycloalkyle, OH, $-(CH_2)_i-C_{3-7}$ cycloalkyle, $C_{1-6}$ alkyle-OH,
- $(CH_2)_i-C_{1-6}$ alkyloxy, $-(CH_2)_i-C_{1-6}$ fluoroalkyle, $-(CH_2)_z-O-C_{1-6}$ fluoroalkyle, $COR_a$, CN, ou $NR_{14}R_{15}$
- $R_8$ et $R_{8'}$ sont identiques ou différents et représentent un groupe $C_{1-6}$ alkyle, $C_{3-7}$ cycloalkyle, $-(CH_2)_f-C_{3-7}$ cycloalkyle ou $-(CH_2)_f-C_{1-6}$ fluoroalkyle.
- $R_9$, $R_{9'}$, $R_{10}$, $R_{11}$, $R_{12}$, $R_{13}$, $R_{14}$ et $R_{15}$ sont identiques ou différents et représentent un atome d'hydrogène, un groupe $C_{1-6}$ alkyle, $C_{3-7}$ cycloalkyle, $-(CH_2)_g-C_{3-7}$ cycloalkyle ou $-(CH_2)_g-C_{1-6}$ fluoroalkyle.
Eventuellement les groupes $R_8$ et $R_9$ peuvent former avec l'atome d'azote qui les porte un hétérocycle tel que : azetidine, pyrolidine, pipéridine, azepane, morpholine ou pipérazine. Eventuellement les groupes $R_{8'}$ et $R_{9'}$ peuvent former avec l'atome d'azote qui les porte un hétérocycle tel que : azetidine, pyrolidine, pipéridine, azepane, morpholine ou pipérazine. Eventuellement les groupes $R_{10}$ et $R_{11}$ peuvent former avec l'atome d'azote qui les porte un hétérocycle tel que : azetidine, pyrolidine, pipéridine, azepane, morpholine ou pipérazine. Eventuellement les groupes $R_{12}$ et $R_{13}$ peuvent former avec l'atome d'azote qui les porte un hétérocycle tel que : azetidine, pyrolidine, pipéridine, azepane, morpholine ou pipérazine. Eventuellement les groupes $R_{14}$ et $R_{15}$ peuvent former avec l'atome d'azote qui les porte un hétérocycle tel que : azetidine, pyrolidine, pipéridine, azepane, morpholine ou pipérazine.
- f, g, i, l, n, r et t sont différents ou identiques et sont égales à 1, 2 ou 3
- j, m, s et v sont différents ou identiques et sont égales à 0, 1 ou 2
- p, q, u, w et z sont différents ou identiques et sont égales à 2, 3 ou 4 ainsi que leurs sels pharmaceutiquement acceptables, solvates ou hydrates et leurs conformères ou rotamères.

**2.** Composés selon la revendication 1 **caractérisés en ce que** :

- X représente un atome de carbone et Y représente un atome de carbone éventuellement substitué par un des groupes tels que définis ci-dessus et préférentiellement un groupe methyle, ethyle, isopropyle, cyclopropyle, $CF_3$, $CONH_2$, $CO_2CH_3$ $CO_2CH_2CH_3$, CN, $NO_2$, $SCH_3$, $SCH_2CH_3$, un atome d'hydrogène, un halogène, $OCF_3$, $OCH_3$, $OCH_2CH_3$ ou $OCH(CH_3)_2$.

**3.** Composés selon l'une des revendications précédentes **caractérisés en ce que** le groupe $R_1$ représente un halogè-

ne, un groupe éthyle, isopropyle, trifluorométhyle, nitrile, nitro, méthoxy, éthoxy, isopropoxy, thiométhyle, thioéthyle, ou thio isopropyle

4. Composés selon la revendication 3 **caractérisés en ce que** le groupe $R_1$ représente un halogène, un groupe methoxy, ethoxy, thiométhyle, thioéthyle ou trifluorométhyle.

5. Composés selon la revendication 1 choisis parmi les composés ci-dessous, leurs sels pharmaceutiquement acceptables, solvates, hydrates, conformères et rotamères :

2-[(6-Methoxy-pyridin-2-ylamino)-methyl]-phenol
2-[(6-Bromo-pyridin-2-ylamino)-methyl]-phenol
2-[(6-Bromo-pyridin-2-ylamino)-methyl]-4-fluoro-phenol
6-(2-Hydroxy-benzylamino)-pyridine-2-carbonitrile
2-[1-(6-Methoxy-pyridin-2-ylamino)-ethyl]-phenol
2-[(6-Trifluoromethyl-pyridin-2-ylamino)-methyl]-phenol
2-[(6-Chloro-pyridin-2-ylamino)-methyl]-phenol
2-[(6-Ethyl-pyridin-2-ylamino)-methyl]-phenol
2-[(6-Ethoxy-pyridin-2-ylamino)-methyl]-phenol
2-[(6-Isopropoxy-pyridin-2-ylamino)-methyl]-phenol
5-Chloro-2-[(6-methoxy-pyridin-2-ylamino)-methyl]-phenol
2-[(2-Trifluoromethyl-pyrimidin-4-ylamino)-methyl]-phenol
2-[(6-Bromo-pyrazin-2-ylamino)-methyl]-phenol
2-[(2-Chloro-pyrimidin-4-ylamino)-methyl]-phenol
2-[(2-Bromo-pyrimidin-4-ylamino)-methyl]-phenol
2-[(2-Chloro-6-methyl-pyrimidin-4-ylamino)-methyl]-phenol
2-[(6-Chloro-4-trifluoromethyl-pyridin-2-ylamino)-methyl]-phenol
2-[(6-Chloro-4-methyl-pyridin-2-ylamino)-methyl]-phenol
2-[(6-Methoxy-pyrazin-2-ylamino)-methyl]-phenol
2-[(2-Methoxy-pyrimidin-4-ylamino)-methyl]-phenol
2-[(2-Methoxy-6-methyl-pyrimidin-4-ylamino)-methyl]-phenol
2-[(6-Methylsulfanyl-pyridin-2-ylamino)-methyl]-phenol
2-[(6-Methanesulfinyl-pyridin-2-ylamino)-methyl]-phenol
2-[(6-Methanesulfonyl-pyridin-2-ylamino)-methyl]-phenol
2-[(6-Methoxy-pyridin-2-ylamino)-methyl]-6-methyl-phenol
2-[(4-Bromo-6-methoxy-pyridin-2-ylamino)-methyl]-phenol
2-[(6-Bromo-2-methoxy-pyrimidin-4-ylamino)-methyl]-phenol
2-[(4-Chloro-6-methoxy-pyridin-2-ylamino)-methyl]-phenol
2-[(6-Bromo-2-methoxy-pyrimidin-4-ylamino)-methyl]-phenol
2-[(4-Bromo-6-methoxy-pyridin-2-ylamino)-methyl]-6-fluoro-phenol
2-[(4-Bromo-6-methoxy-pyridin-2-ylamino)-methyl]-5-fluoro-phenol
2-[(4-Bromo-6-methoxy-pyridin-2-ylamino)-methyl]-3-fluoro-phenol
2-[(4-Bromo-6-methoxy-pyridin-2-ylamino)-methyl]-4-fluoro-phenol
2-[(6-Bromo-2-methoxy-pyrimidin-4-ylamino)-methyl]-4-fluoro-phenol
2-[(4-Chloro-6-methoxy-pyridin-2-ylamino)-methyl]-4-fluoro-phenol
2-[(6-Chloro-2-methoxy-pyrimidin-4-ylamino)-methyl]-4-fluoro-phenol
2-[1-(4-Bromo-6-methoxy-pyridin-2-ylamino)-ethyl]-phenol
2-[1-(4-Bromo-6-methoxy-pyridin-2-ylamino)-propyl]-phenol
2-[1-(6-Bromo-4-methyl-pyridin-2-ylamino)-1-methyl-ethyl]-phenol
2-[1-(4-Bromo-6-methoxy-pyridin-2-ylamino)-propyl]-4-fluoro-phenol
2-[1-(6-Bromo-pyridin-2-ylamino)-propyl]-4-fluoro-phenol
4-Fluoro-2-[(6-methoxy-pyridin-2-ylamino)-methyl]-phenol
4-Fluoro-2-[1-(6-methoxy-pyridin-2-ylamino)-ethyl]-phenol
4-Fluoro-2-[1-(6-methoxy-pyridin-2-ylamino)-propyl]-phenol
2-[(6-Bromo-4-methoxy-pyridin-2-ylamino)-methyl]-phenol
2-[(6-Bromo-4-methyl-pyridin-2-ylamino)-methyl]-phenol
2-[(6-Chloro-4-methoxy-pyridin-2-ylamino)-methyl]-phenol

6. Composés selon l'une des revendications précédentes, pour leur utilisation en tant que médicaments.

7. Utilisation cosmétique d'un composé tel que défini selon l'une des revendications 1 à 5 pour l'hygiène corporelle ou capillaire.

8. Utilisation d'un composé selon l'une quelconque des revendications 1 à 5 pour la fabrication d'un médicament pour prévenir et/ou traiter l'hirsutisme, l'alopécie androgénique, de l'hyperpilosité, les désordres de la glande sébacée tels que l'hyperséborrhée, l'acné, la peau grasse ou la dermite séborrhéique.

9. Utilisation d'un composé selon l'une quelconque des revendications 1 à 5 pour la fabrication d'un médicament pour traiter l'acné.

10. Composés selon l'une quelconque des revendications 1 à 5 pour une utilisation dans la prévention et/ou le traitement de l'hirsutisme, l'alopécie androgénique, l'hyperpilosité, l'hyperséborrhée, l'acné, la peau grasse ou la dermite séborrhéique.

11. Composés selon l'une quelconque des revendications 1 à 5 pour une utilisation dans le traitement de l'acné.

**Patentansprüche**

1. Verbindungen der Formel (I):

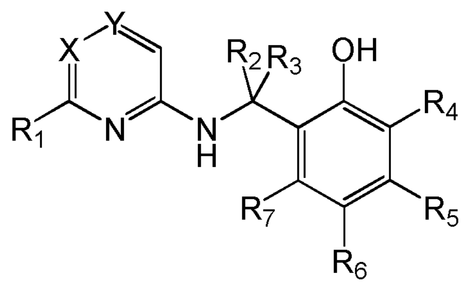

(I)

in der:

- $R_1$ für eine $C_{2-6}$-Alkyl-, $C_{3-7}$-Cycloalkyl-, $C_{1-6}$-Alkyloxy-, $-S(O)_m-C_{1-6}$-Alkyl-, $C_{1-6}$-Fluoralkyl-, $C_{1-6}$-Fluoralkyloxy-, $-(CH_2)_n-C_{3-9}$-Cycloalkyl-, $C_{2-6}$-Alkyl-OH, $-(CH_2)_n-C_{1-6}$-Alkyloxy-, $-(CH_2)_n-C_{1-6}$-Fluoralkyl-, $-(CH_2)_p-O-C_{1-6}$-Fluoralkyl-, $COR_a$, CN, $NO_2$, $NR_8R_9$-Gruppe, ein Halogen, eine Phenyl- oder Heteroaryl-Gruppe steht, enthaltend entweder a) 1 bis 4 Stickstoffatome oder b) ein Sauerstoff- oder Schwefelatom und 1 oder 2 Stickstoffatome. Diese Phenyl- und Heteroaryl-Gruppen können eventuell mit ein bis drei identischen oder verschiedenen Gruppen $R_b$ substituiert sein,
- $R_2$ und $R_3$ identisch oder verschieden sind und für ein Wasserstoffatom oder eine $C_{1-9}$-Alkyl-, $C_{3-9}$-Cycloalkyl-, $C_{1-6}$-Fluoralkyl-, $-(CH_2)_r-C_{3-9}$-Cycloalkyl-, $C_{2-6}$-Alkyl-OH,$-(CH_2)_r-C_{1-6}$-Alkyloxy-, $-(CH_2)_r-C_{3-7}$-Cycloalkyl-, $-(CH_2)_r-C_{1-6}$-Fluoralkyl-, $-(CH_2)_q-O-C_{1-6}$-Fluoralkyl-Gruppe stehen,
eventuell können die Gruppen $R_2$ und $R_3$ mit dem Kohlenstoffatom, das sie trägt, eine $C_{3-9}$-Cycloalkyl-Gruppe oder einen Heterocyclus wie Tetrahydrofuran, Tetrahydropyran, Tetrahydrothiopyran, Tetrahydro-1-oxy-thiopyran oder Tetrahydro-1,1-dioxy-thiopyran bilden,
- $R_4$, $R_5$, $R_6$, $R_7$ identisch oder verschieden sind und entweder für ein Wasserstoffatom oder eine $C_{1-6}$-Alkyl-, $C_{3-7}$-Cycloalkyl-, $C_{1-6}$-Alkyloxy-, $-S(O)_s-C_{1-6}$-Alkyl-, $C_{1-6}$-Fluoralkyl-, $C_{1-6}$-Fluoralkyloxy-, $-(CH_2)_t-C_{3-9}$-Cycloalkyl-, $-(CH_2)_t-C_{3-9}$-Cycloalkyl-, $C_{1-6}$-Alkyl-OH,$-(CH_2)_t-C_{1-6}$-Alkyloxy-, $-(CH_2)_t-C_{1-6}$-Fluoralkyl-, $-(CH_2)_u-O-C_{1-6}$-Fluoralkyl-, $COR_d$, CN, $NR_{8'}R_{9'}$-Gruppe oder ein Halogen oder eine Phenyl- oder Heteroaryl-Gruppe stehen, enthaltend entweder a) 1 bis 4 Stickstoffatome oder b) ein Sauerstoff- oder Schwefelatom und 1 oder 2 Stickstoffatome. Diese Phenyl- und Heteroaryl-Gruppen können eventuell mit ein bis drei identischen oder verschiedenen $R_c$-Gruppen substituiert sein,
- X für CH oder N steht,
- Y entweder für ein Stickstoffatom oder ein Kohlenstoffatom steht, substituiert mit einer $C_{1-6}$-Alkyl-, $C_{3-7}$-Cycloalkyl-, $C_{1-6}$-Alkyloxy-, $-S(O)_v-C_{1-6}$-Alkyl-, $C_{1-6}$-Fluoralkyl-, $C_{1-6}$-Fluoralkyloxy-, $-(CH_2)_l-C_{3-9}$-Cycloalkyl-, $-(CH_2)_l-C_{3-9}$-Cycloalkyl-, $C_{1-6}$-Alkyl-OH,$-(CH_2)_l-C_{1-6}$-Alkyloxy-, $-(CH_2)_l-C_{1-6}$-Fluoralkyl-, $-(CH_2)_w-O-C_{1-6}$-Fluoral-

kyl-, $COR_e$, CN, oder $NR_{10}R_{11}$, $NO_2$ Gruppe, einem Wasserstoffatom oder einem Halogen oder einer Phenyl- oder Heteroaryl-Gruppe, enthaltend entweder a) 1 bis 4 Stickstoffatome oder b) ein Sauerstoff- oder Schwefelatom und 1 oder 2 Stickstoffatome. Diese Phenyl- und Heteroaryl-Gruppen können eventuell mit ein bis drei identischen oder verschiedenen Gruppen $R_b$ substituiert sein

- $R_a$, $R_d$ und $R_e$ identisch oder verschieden sind und für eine $C_{1-6}$-Alkyl-, $C_{1-6}$-Alkyloxy- oder $NR_{12}R_{13}$-Gruppe stehen;
- $R_b$ und $R_c$ identisch oder verschieden sind und für ein Halogen, eine $C_{1-6}$-Alkyl-, $C_{3-7}$-Cycloalkyl-, $C_{1-6}$-Alkyloxy-, $-S(O)_j-C_{1-6}$-Alkyl-, $C_{1-6}$-Fluoralkyl-, $C_{1-6}$-Fluoralkyloxy-,$-(CH_2)_i-C_{3-7}$-Cycloalkyl-, OH, $-(CH_2)_i-C_{3-7}$-Cycloalkyl-, $C_{1-6}$-Alkyl-OH, $-(CH_2)_i-C_{1-6}$-Alkyloxy-, $-(CH_2)_i-C_{1-6}$-Fluoralkyl-, $-(CH_2)_z-O-C_{1-6}$-Fluoralkyl-, $COR_a$, CN, oder $NR_{14}R_{15}$-Gruppe stehen,
- $R_8$ und $R_{8'}$ identisch oder verschieden sind und für eine $C_{1-6}$-Alkyl-, $C_{3-7}$-Cycloalkyl-,$-(CH_2)_f-C_{3-7}$-Cycloalkyl- oder $-(CH_2)_f-C_{3-7}$-Fluoralkyl-Gruppe stehen,
- $R_9$, $R_{9'}$, $R_{10}$, $R_{11}$, $R_{12}$, $R_{13}$, $R_{14}$ und $R_{15}$ identisch oder verschieden sind und für ein Wasserstoffatom, eine $C_{1-6}$-Alkyl-, $C_{3-7}$-Cycloalkyl-, $-(CH_2)_g-C_{3-7}$-Cycloalkyl- oder $-(CH_2)_g-C_{1-6}$-Fluoralkyl-Gruppe stehen.

Eventuell können die Gruppen $R_8$ und $R_9$ mit dem Stickstoffatom, das sie trägt, einen Heterocyclus wie Azetidin, Pyrrolidin, Piperidin, Azepan, Morpholin oder Piperazin bilden. Eventuell können die Gruppen $R_{8'}$ und $R_{9'}$ mit dem Stickstoffatom, das sie trägt, einen Heterocyclus wie Azetidin, Pyrrolidin, Piperidin, Azepan, Morpholin oder Piperazin bilden. Eventuell können die Gruppen $R_{10}$ und $R_{11}$ mit dem Stickstoffatom, das sie trägt, einen Heterocyclus wie Azetidin, Pyrrolidin, Piperidin, Azepan, Morpholin oder Piperazin bilden. Eventuell können die Gruppen $R_{12}$ und $R_{13}$ mit dem Stickstoffatom, das sie trägt, einen Heterocyclus wie Azetidin, Pyrrolidin, Piperidin, Azepan, Morpholin oder Piperazin bilden. Eventuell können die Gruppen $R_{14}$ und $R_{15}$ mit dem Stickstoffatom, das sie trägt, einen Heterocyclus wie Azetidin, Pyrrolidin, Piperidin, Azepan, Morpholin oder Piperazin bilden.

- f, g, i, l, n, r und t verschieden oder gleich sind und gleich 1, 2 oder 3 sind,
- j, m, s und v verschieden oder gleich sind und gleich 0, 1 oder 2 sind,
- p, q, u, w und z verschieden oder gleich sind und gleich 2, 3 oder 4 sind,

sowie ihre pharmazeutisch verträglichen Salze, Solvate oder Hydrate und ihre Konformere und Rotamere.

**2.** Verbindungen gemäß Anspruch 1, **dadurch gekennzeichnet, dass**

- X für ein Kohlenstoffatom steht und Y für ein Kohlenstoffatom steht, eventuell substituiert mit einer der oben definierten Gruppen und vorzugsweise mit einer Methyl-, Ethyl-, Isopropyl-, Cyclopropyl-, $CF_3$, $CONH_2$, $CO_2CH_3$, $CO_2CH_2CH_3$, CN, $NO_2$, $SCH_3$, $SCH_2CH_3$-Gruppe, einem Wasserstoffatom, einem Halogen, einer $OCF_3$, $OCH_3$, $OCH_2CH_3$ oder $OCH(CH_3)_2$-Gruppe.

**3.** Verbindungen gemäß einem der vorhergehenden Ansprüche, **dadurch gekennzeichnet, dass** die Gruppe $R_1$ für ein Halogen, eine Ethyl-, Isopropyl-, Trifluormethyl-, Nitril-, Nitro-, Methoxy-, Ethoxy-, Isopropoxy-, Thiomethyl-, Thioethyl- oder Thioisopropyl-Gruppe steht.

**4.** Verbindungen gemäß Anspruch 3, **dadurch gekennzeichnet, dass** die Gruppe $R_1$ für ein Halogen, eine Methoxy-, Ethoxy-, Thiomethyl-, Thioethyl- oder Trifluormethyl-Gruppe steht.

**5.** Verbindungen gemäß Anspruch 1, ausgewählt aus nachstehenden Verbindungen, ihren pharmazeutisch verträglichen Salzen, Solvaten, Hydraten, Konformeren und Rotameren:

2-[(6-Methoxy-pyridin-2-ylamino)-methyl]-phenol
2-[(6-Brom-pyridin-2-ylamino)-methyl]-phenol
2-[(6-Brom-pyridin-2-ylamino)-methyl]-4-fluor-phenol
6-(2-Hydroxy-benzylamino)-pyridin-2-carbonitril
2-[1-(6-Methoxy-pyridin-2-ylamino)-ethyl]-phenol
2-[(6-Trifluormethyl-pyridin-2-ylamino)-methyl]-phenol
2-[(6-Chlor-pyridin-2-ylamino)-methyl]-phenol
2-[(6-Ethyl-pyridin-2-ylamino)-methyl]-phenol
2-[(6-Ethoxy-pyridin-2-ylamino)-methyl]-phenol
2-[(6-Isopropoxy-pyridin-2-ylamino)-methyl]-phenol
5-Chlor-2-[(6-methoxy-pyridin-2-ylamino)-methyl]-phenol
2-[(2-Trifluormethyl-pyrimidin-4-ylamino)-methyl]-phenol
2-[(6-Brom-pyrazin-2-ylamino)-methyl]-phenol

2-[(2-Chlor-pyrimidin-4-ylamino)-methyl]-phenol
2-[(2-Brom-pyrimidin-4-ylamino)-methyl]-phenol
2-[(2-Chlor-6-methyl-pyrimidin-4-ylamino)-methyl]-phenol
2-[(6-Chlor-4-trifluormethyl-pyridin-2-ylamino)-methyl]-phenol
2-[(6-Chlor-4-methyl-pyridin-2-ylamino)-methyl]-phenol
2-[(6-Methoxy-pyrazin-2-ylamino)-methyl]-phenol
2-[(6-Methoxy-pyrimidin-4-ylamino)-methyl]-phenol
2-[(2-Methoxy-6-methyl-pyrimidin-4-ylamino)-methyl]-phenol
2-[(6-Methylsulfanyl-pyridin-2-ylamino)-methyl]-phenol
2-[(6-Methansulfinyl-pyridin-2-ylamino)-methyl]-phenol
2-[(6-Methansulfonyl-pyridin-2-ylamino)-methyl]-phenol
2-[(6-Methoxy-pyridin-2-ylamino)-methyl]-6-methyl-phenol
2-[(4-Brom-6-methoxy-pyridin-2-ylamino)-methyl]-phenol
2-[(6-Brom-2-methoxy-pyrimidin-4-ylamino)-methyl]-phenol
2-[(4-Chlor-6-methoxy-pyridin-2-ylamino)-methyl]-phenol
2-[(6-Brom-2-methoxy-pyrimidin-4-ylamino)-methyl]-phenol
2-[(4-Brom-6-methoxy-pyridin-2-ylamino)-methyl]-6-fluor-phenol
2-[(4-Brom-6-methoxy-pyridin-2-ylamino)-methyl]-5-fluor-phenol
2-[(4-Brom-6-methoxy-pyridin-2-ylamino)-methyl]-3-fluor-phenol
2-[(4-Brom-6-methoxy-pyridin-2-ylamino)-methyl]-4-fluor-phenol
2-[(6-Brom-2-methoxy-pyrimidin-4-ylamino)-methyl]-4-fluor-phenol
2-[(4-Chlor-6-methoxy-pyridin-2-ylamino)-methyl]-4-fluor-phenol
2-[(6-Chlor-2-methoxy-pyrimidin-4-ylamino)-methyl]-4-fluor-phenol
2-[1-(4-Brom-6-methoxy-pyridin-2-ylamino)-ethyl]-phenol
2-[1-(4-Brom-6-methoxy-pyridin-2-ylamino)-propyl]-phenol
2-[1-(6-Brom-4-methyl-pyridin-2-ylamino)-1-methyl-ethyl]-phenol
2-[1-(4-Brom-6-methoxy-pyridin-2-ylamino)-propyl]-4-fluor-phenol
2-[1-(6-Brom-pyridin-2-ylamino)-propyl]-4-fluor-phenol
4-Fluor-2-[(6-methoxy-pyridin-2-ylamino)-methyl]-phenol
4-Fluor-2-[1-(6-methoxy-pyridin-2-ylamino)-ethyl]-phenol
4-Fluor-2-[1-(6-methoxy-pyridin-2-ylamino)-propyl]-phenol
2-[(6-Brom-4-methoxy-pyridin-2-ylamino)-methyl]-phenol
2-[(6-Brom-4-methyl-pyridin-2-ylamino)-methyl]-phenol
2-[(6-Chlor-4-methoxy-pyridin-2-ylamino)-methyl]-phenol

6. Verbindungen gemäß einem der vorhergehenden Ansprüche als Medikamente.

7. Kosmetische Verwendung einer Verbindung, definiert gemäß einem der Ansprüche 1 bis 5, für die Körper- oder Haarpflege.

8. Verwendung einer Verbindung gemäß einem der Ansprüche 1 bis 5 für die Herstellung eines Medikaments zur Prävention und/oder Behandlung von Hirsutismus, Alopecia androgenetica, übermäßiger Behaarung, Störungen der Talgdrüse wie Hyperseborrhö, Akne, fettiger Haut oder Dermatitis seborrhoica.

9. Verwendung einer Verbindung gemäß einem der Ansprüche 1 bis 5 für die Herstellung eines Medikaments zur Behandlung der Akne.

10. Verbindungen gemäß einem der Ansprüche 1 bis 5 für eine Verwendung in der Prävention und/oder Behandlung von Hirsutismus, Alopecia androgenetica, übermäßiger Behaarung, Hyperseborrhö, Akne, fettiger Haut oder Dermatitis seborrhoica.

11. Verbindungen gemäß einem der Ansprüche 1 bis 5 für eine Verwendung in der Behandlung der Akne.

## Claims

1. Compounds of formula (I):

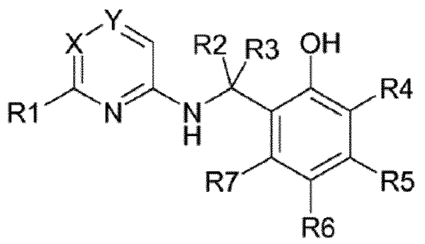

wherein:

- $R_1$ represents a $C_{2-6}$ alkyl, $C_{3-7}$ cycloalkyl, $C_{1-6}$ alkyloxy,-$S(O)_m$-$C_{1-6}$ alkyl, $C_{1-6}$ fluoroalkyl, $C_{1-6}$ fluoroalkyloxy, -$(CH_2)_n$-$C_{3-9}$ cycloalkyl, $C_{2-6}$ alkyl-OH, -$(CH_2)_n$-$C_{1-6}$ alkyloxy, -$(CH_2)_n$-$C_{1-6}$ fluoroalkyl, -$(CH_2)_p$-O-$C_{1-6}$ fluoroalkyl, $COR_a$, CN, $NO_2$, $NR_8R_9$, halogen, phenyl or heteroaryl group containing either a) 1 to 4 nitrogen atoms or b) an oxygen or sulfur atom and 1 or 2 carbon nitrogen. These phenyl and heteroaryl groups can optionally be substituted by one to three identical or different groups $R_b$,
- $R_2$ and $R_3$ are identical or different and represent a hydrogen atom or a $C_{1-9}$ alkyl, $C_{3-9}$ cycloalkyl, $C_{1-6}$ fluoroalkyl, -$(CH_2)_r$-$C_{3-9}$ cycloalkyl, -$C_{2-6}$-alkyl-OH, -$(CH_2)_r$-$C_{1-6}$ alkyloxy, -$(CH_2)_r$-$C_{3-7}$ cycloalkyl, -$(CH_2)$-$C_{1-6}$ fluoroalkyl, -$(CH_2)_q$-O-$C_{1-6}$ fluoroalkyl Optionally $R_2$ and $R_3$ may form with the carbon atom carrying them, a $C_{3-9}$ cycloalkyl group or a heterocycle such as tetrahydrofuran, tetrahydropyran, tetrahydrothiopyran, tetrahydro-1-oxy-thiopyran or tetrahydro-1,1 dioxy thiopyran.
- $R_4$, $R_5$, $R_6$, $R_7$ are identical or different and represent either hydrogen or a $C_{1-6}$ alkyl group, $C_{3-7}$ cycloalkyl, $C_{1-6}$ alkyloxy, -$S(O)_s$-$C_{1-6}$ alkyl, $C_{1-6}$ fluoroalkyl, $C_{1-6}$ fluoroalkyloxy, -$(CH_2)_t$-$C_{3-9}$ cycloalkyl, -$(CH_2)_t$-$C_{3-9}$ cycloalkyl, $C_{1-6}$ alkyl-OH, -$(CH_2)_t$-$C_{1-6}$ alkyloxy, -$(CH_2)_t$-$C_{1-6}$ fluoroalkyl, -$(CH_2)_u$-O-$C_{1-6}$ fluoroalkyl, $COR_d$, CN, $NR_{8'}R_{9'}$, or a halogen or phenyl or heteroaryl group containing either a) from 1 to 4 carbon nitrogen or b) an oxygen atom or sulfur atom and 1 or 2 nitrogen atoms. These phenyl and heteroaryl groups may optionally be substituted by one to three identical or different groups $R_c$
- X represents CH or N
- Y represents either a nitrogen atom or a carbon atom substituted by a $C_{1-6}$ alkyl, $C_{3-7}$ cycloalkyl, $C_{1-6}$ alkyloxy, -$S(O)_v$-$C_{1-6}$ alkyl, $C_{1-6}$ fluoroalkyl, $C_{1-6}$ fluoroalkyloxy, -$(CH_2)_i$-$C_{3-9}$ cycloalkyl, -$(CH_2)_i$-$C_{3-9}$ cycloalkyl, $C_{1-6}$ alkyl-OH, -$(CH_2)_i$-$C_{1-6}$ alkyloxy, -$(CH_2)_i$-$C_{1-6}$ fluoroalkyl, -$(CH_2)_w$-O-$C_{1-6}$ fluoroalkyl, $COR_e$, CN, $NR_{10}R_{11}$, $NO_2$, a hydrogen or halogen atom or a phenyl or heteroaryl group containing either a) 1 to 4 nitrogen atoms or b) one oxygen atom or sulfur atom and 1 or 2 nitrogen atoms. These groups phenyl and heteroaryl can optionally be substituted by one to three identical or different groups Rb
- $R_a$, $R_d$ and $R_e$ are identical or different and represent a $C_{1-6}$ alkyl, $C_{1-6}$ alkyloxy or $NR_{12}R_{13}$ group,
- $R_b$ and $R_c$ are identical or different and represent halogen, $C_{1-6}$ alkyl, $C_{3-7}$ cycloalkyl, $C_{1-6}$ alkyloxy, -$S(O)_j$-$C_{1-6}$ alkyl, $C_{1-6}$ fluoroalkyl, $C_{1-6}$ fluoroalkyloxy, -$(CH_2)_i$-$C_{3-7}$ cycloalkyl, OH, -$(CH_2)_i$-$C_{3-7}$ cycloalkyl, $C_{1-6}$ alkyl-OH, -$(CH_2)_i$-$C_{1-6}$ alkyloxy, -(CH2)$_i$-$C_{1-6}$ fluoroalkyl, -$(CH_2)_z$-O-$C_{1-6}$ fluoroalkyl, $COR_a$, CN, or $NR_{14}R_{15}$
- $R_8$ and $R_{8'}$ are identical or different and represent a $C_{1-6}$ alkyl, $C_{3-7}$ cycloalkyl, -$(CH_2)_f$-$C_{3-7}$ cycloalkyl or -$(CH_2)_f$-$C_{1-6}$ fluoroalkyl.
- $R_9$, $R_{9'}$, $R_{10}$, $R_{11}$, $R_{12}$, $R_{13}$, $R_{14}$ and $R_{15}$ are identical or different and represent a hydrogen atom, a $C_{1-6}$ alkyl, $C_{3-7}$ cycloalkyl, -$(CH_2)_g$-$C_{3-7}$ cycloalkyl or -$(CH_2)_g$-$C_{1-6}$ fluoroalkyl. Optionally the groups $R_8$ and $R_9$ may form, with the nitrogen atom which carries them a heterocycle as: azetidine, pyrrolidine, piperidine, azepane, morpholine or piperazine. Optionally the groups $R_{8'}$ and $R_{9'}$ may form, with the nitrogen atom which carries them a heterocycle as: azetidine, pyrrolidine, piperidine, azepane, morpholine or piperazine. Optionally the groups $R_{10}$ and $R_{11}$ may form with the nitrogen atom which carries them a heterocycle as: azetidine, pyrrolidine, piperidine, azepane, morpholine or piperazine. Optionally the groups $R_{12}$ and $R_{13}$ may form with the nitrogen atom which carries them a heterocycle as: azetidine, pyrrolidine, piperidine, azepane, morpholine or piperazine. Optionally the $R_{14}$ and $R_{15}$ groups may form, with the nitrogen atom which carries them a heterocycle as: azetidine, pyrrolidine, piperidine, azepane, morpholine or piperazine.
- f, g, i, l, n, r and t are identical or different and are 1, 2 or 3
- j, m, s and v are identical or different and are 0, 1 or 2
- p, q, u, w and z are identical or different and are equal to 2, 3 or 4

and their pharmaceutically acceptable salts, hydrates or solvates and their conformers or rotamers.

2. Compounds according to claim 1 **characterized in that**:

- X represents a carbon atom and Y represents a carbon atom optionally substituted by a group as defined above and preferably a methyl, ethyl, isopropyl, cyclopropyl, $CF_3$, $CONH_2$, $CO_2CH_3$, $CO_2CH_2CH_3$, CN, $NO_2$ $SCH_3$, $SCH_2CH_3$, hydrogen, halogen, $OCF_3$, $OCH_3$, $OCH_2CH_3$, or $OCH(CH_3)_2$.

3. Compounds according to any one of the preceding claims, **characterized in that** the group $R_1$ is halogen, ethyl, isopropyl, trifluoromethyl, nitrile, nitro, methoxy, ethoxy, isopropoxy, thiomethyl, thioethyl, or thio isopropyl.

4. Compounds according to claim 3, **characterized in that** the group $R_1$ represents a halogen, methoxy, ethoxy, thiomethyl, thioethyl or trifluoromethyl.

5. Compounds according to claim 1 selected from the following compounds, their pharmaceutically acceptable salts, solvates, hydrates, conformers and rotamers:

2-[(6-Methoxy-pyridin-2-ylamino)-methyl]-phenol
2-[(6-Bromo-pyridin-2-ylamino)-methyl]-phenol
2-[(6-Bromo-pyridin-2-ylamino)-methyl]-4-fluoro-phenol
6-(2-Hydroxy-benzylamino)-pyridine-2-carbonitrile
2-[1-(6-Methoxy-pyridin-2-ylamino)-ethyl]-phenol
2-[(6-Trifluoromethyl-pyridin-2-ylamino)-methyl]-phenol
2-[(6-Chloro-pyridin-2-ylamino)-methyl]-phenol
2-[(6-Ethyl-pyridin-2-ylamino)-methyl]-phenol
2-[(6-Ethoxy-pyridin-2-ylamino)-methyl]-phenol
2-[(6-Isopropoxy-pyridin-2-ylamino)-methyl]-phenol
5-Chloro-2-[(6-methoxy-pyridin-2-ylamino)-methyl]-phenol
2-[(2-Trifluoromethyl-pyrimidin-4-ylamino)-methyl]-phenol
2-[(6-Bromo-pyrazin-2-ylamino)-methyl]-phenol
2-[(2-Chloro-pyrimidin-4-ylamino)-methyl]-phenol
2-[(2-Bromo-pyrimidin-4-ylamino)-methyl]-phenol
2-[(2-Chloro-6-methyl-pyrimidin-4-ylamino)-methyl]-phenol
2-[(6-Chloro-4-trifluoromethyl-pyridin-2-ylamino)-methyl]-phenol
2-[(6-Chloro-4-methyl-pyridin-2-ylamino)-methyl]-phenol
2-[(6-Methoxy-pyrazin-2-ylamino)-methyl]-phenol
2-[(2-Methoxy-pyrimidin-4-ylamino)-methyl]-phenol
2-[(2-Methoxy-6-methyl-pyrimidin-4-ylamino)-methyl]-phenol
2-[(6-methylsulfanyl-pyridin-2-ylamino)-methyl]-phenol
2-[(6-Methanesulfinyl-pyridin-2-ylamino)-methyl]-phenol
2-[(6-methanesulfonyl-pyridin-2-ylamino)-methyl]-phenol
2-[(6-Methoxy-pyridin-2-ylamino)-methyl]-6-methyl-phenol
2-[(4-Bromo-6-methoxy-pyridin-2-ylamino)-methyl]-phenol
2-[(6-Bromo-2-methoxy-pyrimidin-4-ylamino)-methyl]-phenol
2-[(4-Chloro-6-methoxy-pyridin-2-ylamino)-methyl]-phenol
2-[(6-Bromo-2-methoxy-pyrimidin-4-ylamino)-methyl]-phenol
2-[(4-Bromo-6-methoxy-pyridin-2-ylamino)-methyl]-6-fluoro-phenol
2-[(4-Bromo-6-methoxy-pyridin-2-ylamino)-methyl]-5-fluoro-phenol
2-[(4-Bromo-6-methoxy-pyridin-2-ylamino)-methyl]-3-fluoro-phenol
2-[(4-Bromo-6-methoxy-pyridin-2-ylamino)-methyl]-4-fluoro-phenol
2-[(6-Bromo-2-methoxy-pyrimidin-4-ylamino)-methyl]-4-fluoro-phenol
2-[(4-Chloro-6-methoxy-pyridin-2-ylamino)-methyl]-4-fluoro-phenol
2-[(6-Chloro-2-methoxy-pyrimidin-4-ylamino)-methyl]-4-fluoro-phenol
2-[1-(4-Bromo-6-methoxy-pyridin-2-ylamino)-ethyl]-phenol
2-[1-(4-Bromo-6-methoxy-pyridin-2-ylamino)-propyl]-phenol
2-[1-(6-Bromo-4-methyl-pyridin-2-ylamino)-1-methyl-ethyl]-phenol
2-[1-(4-Bromo-6-methoxy-pyridin-2-ylamino)-propyl]-4-fluoro-phenol
2-[1-(6-Bromo-pyridin-2-ylamino)-propyl]-4-fluoro-phenol
4-Fluoro-2-[(6-methoxy-pyridin-2-ylamino)-methyl]-phenol
4-Fluoro-2-[1-(6-methoxy-pyridin-2-ylamino)-ethyl]-phenol
4-Fluoro-2-[1-(6-methoxy-pyridin-2-ylamino)-propyl]-phenol
2-[(6-Bromo-4-methoxy-pyridin-2-ylamino)-methyl]-phenol

2-[(6-Bromo-4-methyl-pyridin-2-ylamino)-methyl]-phenol
2-[(6-Chloro-4-methoxy-pyridin-2-ylamino)-methyl]-phenol

**6.** Compounds according to any one of the preceding claims, for use as medicaments.

**7.** Cosmetic use of a compound as defined according to any one of claims 1 to 5, for body or hair hygiene.

**8.** Use of a compound according to any one of claims 1 to 5 for the manufacture of a medicament for preventing and/or treating hirsutism, androgenic alopecia, excessive hair, disorders of the Sebaceous gland such as the hyperseborrhoea of acne, oily skin or seborrheic dermatitis.

**9.** Use of a compound according to any one of claims 1 to 5 for the manufacture of a medicament for treating acne.

**10.** Compounds according to any one of claims 1 to 5 for use for preventing and/or treating hirsutism, androgenic alopecia, excessive hair, hyperseborrhoea, acne, oily skin or seborrheic dermatitis.

**11.** Compounds according to any one of claims 1 to 5 for use for treating acne.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 580459 A
- WO 200542464 A
- US 4578390 A
- WO 2004052868 A


### Littérature non-brevet citée dans la description


- Handbook of Pharmaceutical Salts : Properties, Selection and Use de Stahl et Wermuth. Wiley-VCH, 2002
- *Org. Pro. R. & D.,* 2006, 971-1031
- Protective Groups in Organic Chemistry. Plenum Press, 1973
- Protective Groups in Organic Synthesis. John Wiley et Sons, 1991
- **KOCIENSKI P.J.** Protecting Groups. Georg Thieme Verlag, 1994
- **MONOD.** *J. Motecular Biology,* 1965, vol. 12 (1), 88-118